(19) Europäisches Patentamt
European Patent Office
Office européen des brevets

(11) **EP 2 990 470 A1**

(12) **EUROPÄISCHE PATENTANMELDUNG**

(43) Veröffentlichungstag:
**02.03.2016 Patentblatt 2016/09**

(51) Int Cl.:
***C11C 3/00*** *(2006.01)*

(21) Anmeldenummer: **14182190.0**

(22) Anmeldetag: **26.08.2014**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(71) Anmelder: **Evonik Degussa GmbH**
**45128 Essen (DE)**

(72) Erfinder:
• **Woldt, Benjamin**
**44892 Bochum (DE)**
• **Boeck, Florian Sebastian**
**48143 Münster (DE)**
• **Gevers, Andreas**
**46240 Bottrop (DE)**
• **Graß, Michael**
**45721 Haltern am See (DE)**

(54) **Gemische epoxidierter Fettsäureester**

(57) Gemische umfassend epoxidierte Fettsäureester, welche einen geringeren Massenanteil an Fettsäureketten-haltigen Verbindungen enthalten, die in der Fettsäurekette keine funktionellen Gruppen einschließlich Mehrfachbindungen aufweisen, als die Gemische der Fettsäurekettenhaltigen Verbindungen, aus denen die Gemische umfassend epoxidierte Fettsäureester hergestellt wurden, können gezielt hergestellt und als Weichmacher eingesetzt werden.

EP 2 990 470 A1

**Beschreibung**

Die vorliegende Erfindung betrifft Gemische umfassend epoxidierte Fettsäureester, Verfahren zur Herstellung solcher Gemische, Mittel enthaltend entsprechende Gemische sowie die Verwendung der Gemische als Weichmacher für Polymere.

Epoxidierte Fettsäureester können als Weichmacher für Polymere, beispielsweise PVC, eingesetzt werden. Im Stand der Technik ist beispielsweise in den Dokumenten WO 01/98404 A2, WO 2011/072346 A1, US 2002/0013396 A1, GB 805,252 B und US 4,486,561 B beschrieben, epoxidierte Fettsäureester natürlichen Ursprungs als Weichmacher zu verwenden. Als Weichmacher finden in diesen Dokumenten die aus Veresterung und Epoxidierung von Fettsäuren direkt resultierenden Produkte Anwendung.

Die in dem Dokument WO 2013/003225 A2 beschriebenen epoxidierten Fettsäureester werden vor dem Einsatz als Weichmacher einem zusätzlichen Aufarbeitungsschritt unterworfen. Dazu wird der Anteil an epoxidierten Linolensäure-Resten gegenüber dem Anteil an epoxidierten Linolsäure-Resten in dem als Weichmacher eingesetzten Gemisch der epoxidierten Fettsäureester verringert, was in einem Weichmacher mit einen geringeren Anteil an "Unreinheiten" resultiert, der in Medizinprodukten eingesetzt werden kann.

Der vorliegenden Erfindung lag die Aufgabe zugrunde, neue Weichmacher bereitzustellen, welche gute, vorzugsweise verbesserte Weichmachereigenschaften aufweisen. Vorteilhafterweise sollten diese Weichmacher auf natürlich vorkommenden Ölen basieren.

Die Aufgabe wird gelöst durch Gemische epoxidierter Fettsäureester, in welchen der Anteil der Fettsäureketten-haltigen Verbindungen, welche keine funktionellen Gruppen einschließlich Mehrfachbindungen aufweisen, verringert wurde.

Gegenstand der Erfindung ist ein Gemisch umfassend epoxidierte Fettsäureester, welches einen geringeren Massenanteil an Fettsäureketten-haltigen Verbindungen enthält, die in der Fettsäurekette keine funktionellen Gruppen einschließlich Mehrfachbindungen aufweisen, als das Gemisch der Fettsäureketten-haltigen Verbindungen, aus denen das Gemisch umfassend epoxidierte Fettsäureester hergestellt wurde. Die Massenanteile basieren jeweils auf der Gesamtheit aller Fettsäureketten-haltigen Verbindungen.

Unter Fettsäureestern werden im Rahmen dieser Erfindung Veresterungsprodukte (Ester) von Fettsäuren mit einwertigen Alkoholen verstanden. Dementsprechend bestehen Fettsäureester aus einem Fettsäurerest, der auch Fettsäurekette genannt wird, und einem Alkoholatrest.

Fettsäureketten-haltige Verbindungen, welche in der Fettsäurekette (das heißt neben der Ester- oder der Säurefunktion) keine weiteren funktionellen Gruppen einschließlich Mehrfachbindungen enthalten, werden im Folgenden vereinfacht als "gesättigte Verbindungen" oder "gesättigt" bezeichnet.

Das Gemisch umfassend epoxidierte Fettsäureester gemäß obigem Gegenstand wird im Folgenden auch vereinfacht als "erfindungsgemäßes" Gemisch oder auch "abgereichertes" Gemisch bezeichnet.

Ein Gemisch umfassend epoxidierte Fettsäureester, welches einen vergleichbaren (denselben) Massenanteil an Fettsäureketten-haltigen Verbindungen enthält, die in der Fettsäurekette keine funktionellen Gruppen einschließlich Mehrfachbindungen aufweisen, wie das Gemisch der Fettsäureketten-haltigen Verbindungen, aus denen das Gemisch umfassend epoxidierte Fettsäureester hergestellt wurde, wird im Folgenden als "nicht-abgereichertes" Gemisch bezeichnet. Die Veränderung der Massenanteile, welche ausschließlich auf der Epoxidierung von Doppelbindungen beruht, wird bei diesen Überlegungen nicht betrachtet. Beispielsweise ist ein Gemisch von Fettsäureestern, welches dieselbe Menge an "gesättigten" Verbindungen enthält wie das natürliche Öl, aus dem das Gemisch hergestellt wurde, ein "nicht-abgereichertes" Gemisch. Ein "nicht-abgereichertes" Gemisch hat bei seiner Herstellung keinen Verfahrensschritt durchlaufen, welcher darauf gerichtet war, den Anteil an Fettsäurekettenhaltigen Verbindungen, welche in der Fettsäurekette (neben der Ester- oder der Säurefunktion) keine weiteren funktionellen Gruppen einschließlich Mehrfachbindungen enthalten, zu verringern.

Bevorzugt wurde das Gemisch der Fettsäureketten-haltigen Verbindungen, aus denen das Gemisch umfassend epoxidierte Fettsäureester hergestellt wurde, aus einem pflanzlichen Öl oder aus einem Gemisch mehrerer pflanzlicher Öle gewonnen, wobei das Öl oder das Gemisch der Öle vorzugsweise einen (durchschnittlichen) Doppelbindungsanteil pro Fettsäurekette - ermittelt durch In-Relationsetzen von $^1$H-NMR-Signalen - aufweist, welcher im Bereich von 0,8 bis 2,5, bevorzugt von 1,0 bis 2,0 und insbesondere von 1,2 bis 1,6 liegt. Die Methode zur Bestimmung des Doppelbindungsanteiles ist im experimentellen Teil beschrieben.

Das pflanzliche Öl oder das Gemisch mehrerer pflanzlicher Öle enthält mit Vorzug eines bzw. mehrere Öle aus der Gruppe Sojaöl, Leinöl, Sonnenblumenöl, Distelöl, Rapsöl und Tallöl.

Das Gemisch der Fettsäureketten-haltigen Verbindungen, aus denen das Gemisch umfassend epoxidierte Fettsäureester hergestellt wurde, ist vorzugsweise ein Fettsäuregemisch oder ein Fettsäureestergemisch.

Ist in einer Ausführungsform der vorliegenden Erfindung das Gemisch der Fettsäurekettenhaltigen Verbindungen, aus denen das erfindungsgemäße Gemisch umfassend epoxidierte Fettsäureester hergestellt wurde, ein Fettsäuregemisch, dann enthält das erfindungsgemäße Gemisch umfassend epoxidierte Fettsäureester einen geringeren Massenanteil an Fettsäureestern, die in der Fettsäurekette keine funktionellen Gruppen einschließlich Mehrfachbindungen aufweisen, als das Gemisch der Fettsäuren, aus denen das Gemisch epoxidierter Fettsäureester hergestellt wurde, Fettsäuren enthält, welche in der Fettsäurekette keine funktionellen Gruppen einschließlich Mehrfachbindungen aufweisen. Hierbei basieren die Massenanteile stets auf der Gesamtheit aller Fettsäureketten-haltigen Komponenten. Vereinfacht ausgedrückt: Wurde das erfindungsgemäße Gemisch umfassend epoxidierte Fettsäureester aus Fettsäuren hergestellt, dann ist in dem erfindungsgemäßen Gemisch der Anteil der gesättigten Fettsäureester geringer als der Anteil der gesättigten Fettsäuren in dem zur Herstellung des erfindungsgemäßen Gemisches eingesetzten Fettsäuregemisch, jeweils basierend auf der Gesamtheit aller Fettsäureketten-haltigen Komponenten.

Ist in einer anderen Ausführungsform der vorliegenden Erfindung das Gemisch der Fettsäureketten-haltigen Verbindungen, aus denen das erfindungsgemäße Gemisch umfassend epoxidierte Fettsäureester hergestellt wurde, ein Fettsäureestergemisch, dann enthält das erfindungsgemäße Gemisch umfassend epoxidierte Fettsäureester einen geringeren Massenanteil an Fettsäureestern, die in der Fettsäurekette keine funktionellen Gruppen einschließlich Mehrfachbindungen aufweisen, als das Gemisch der Fettsäureester, aus denen das Gemisch epoxidierter Fettsäureester hergestellt wurde. Hierbei basieren die Massenanteile stets auf der Gesamtheit aller Fettsäureketten-haltigen Komponenten. Vereinfacht ausgedrückt: Wurde das erfindungsgemäße Gemisch umfassend epoxidierte Fettsäureester aus Fettsäureestern hergestellt, dann ist in dem erfindungsgemäßen Gemisch der Anteil der gesättigten Fettsäureester geringer als der Anteil der gesättigten Fettsäureester in dem zur Herstellung des erfindungsgemäßen Gemisches eingesetzten Fettsäureestergemisch, jeweils basierend auf der Gesamtheit aller Fettsäureketten-haltigen Komponenten.

In einer bevorzugten Ausführungsform ist in dem erfindungsgemäßen Gemisch umfassend epoxidierte Fettsäureester der Massenanteil der Fettsäureketten-haltigen Verbindungen, welche in der Fettsäurekette keine funktionellen Gruppen einschließlich Mehrfachbindungen aufweisen, im Vergleich zu dem Gemisch der Fettsäureketten-haltigen Verbindungen, aus denen das Gemisch umfassend epoxidierte Fettsäureester hergestellt wurde, um mindestens 20 %, vorzugsweise um mindestens 40 % mit Vorzug dazu um mindestens 60 % und insbesondere um mindestens 80 % verringert.

Die prozentuale Verringerung lässt sich wie folgt berechnen:

$$\text{Verringerung } [\%] = \frac{\text{"gesättigte Verbindungen" im Edukt} - \text{"gesättigte Verbindungen" im erfindungsgemäßen Gemisch}}{\text{"gesättigte Verbindungen" im Edukt}} \cdot 100$$

hierbei ist

- "gesättigte Verbindungen" im Edukt = Anteil der Fettsäureketten-haltigen Verbindungen, welche in der Fettsäurekette (das heißt neben der Ester- oder der Säurefunktion) keine weiteren funktionellen Gruppen einschließlich Mehrfachbindungen enthalten, in dem Gemisch der Fettsäureketten-haltigen Verbindungen, aus denen das Gemisch umfassend epoxidierte Fettsäureester hergestellt wurde in Massen-%, basierend auf der Gesamtheit aller Fettsäureketten-haltigen Produkte

- "gesättigte Verbindungen" im erfindungsgemäßen Gemisch = Menge der Fettsäureketten-haltigen Verbindungen, welche in der Fettsäurekette (das heißt neben der Ester- oder Säurefunktion) keine weiteren funktionellen Gruppen einschließlich Mehrfachbindungen enthalten, in dem erfindungsgemäßen Gemisch, basierend auf der Gesamtheit aller Fettsäureketten-haltigen Produkte

Ein bevorzugter Gegenstand der vorliegenden Erfindung ist ein Gemisch umfassend epoxidierte Fettsäureester, in welchem der Massenanteil an Fettsäureketten-haltigen Verbindungen welche in der Fettsäurekette keine funktionellen Gruppen einschließlich Mehrfachbindungen aufweisen, im Vergleich zu dem Gemisch der Fettsäureketten-haltigen Verbindungen, aus denen das Gemisch umfassend epoxidierte Fettsäureester hergestellt wurde, um mindestens 20 %, vorzugsweise um mindestens 40 % mit Vorzug dazu um mindestens 60 % und insbesondere um mindestens 80 % verringert ist.

In dem Gemisch umfassend epoxidierte Fettsäureester ist mit Vorzug die mittlere Anzahl an Epoxidgruppen pro Fettsäurekette - ermittelt durch In-Relationsetzen von $^1$H-NMR-Signalen - größer als 1,0, bevorzugt größer als 1,2 und insbesondere größer als 1,4. Die Methode zur Bestimmung der mittleren Anzahl der Epoxidgruppen ist im experimentellen Teil beschrieben.

Wurde das Gemisch der Fettsäureketten-haltigen Verbindungen, aus denen das Gemisch umfassend epoxidierte Fettsäureester hergestellt wurde, aus Leinöl gewonnen, so ist die mittlere Anzahl an Epoxidgruppen pro Fettsäurekette- ermittelt durch In-Relationsetzen von $^1$H-NMR-Signalen - bevorzugt größer als 1,4, besonders bevorzugt größer als 1,6 und insbesondere größer als 1,8.

Die Alkoholatreste der Fettsäureester in dem erfindungsgemäßen Gemisch umfassend epoxidierte Fettsäureester sind vorzugsweise ausgewählt aus Alkoholatresten, welche 1 bis 20, vorzugsweise 2 bis 15 und insbesondere 4 bis 11 Kohlenstoffatome enthalten, wobei die Alkoholatreste bevorzugt (neben der Alkoholatfunktion) keine weiteren funktionellen Gruppen einschließlich Mehrfachbindungen aufweisen. Bevorzugt sind die Alkoholatreste der Fettsäureester in dem erfindungsgemäßen Gemisch ausgewählt aus Methyl-, Ethyl, Propyl-, Butyl-, *tert*-Butyl, *iso*-Butyl, 2-Methylbutyl-, 3-Methylbutyl-, *n*-Pentyl-, Hexyl-, Heptyl-, *iso*-Heptyl-, Octyl-, *iso*-Octyl-, 2-Ethylhexyl-, Nonyl-, *n*-Nonyl-, *iso*-Nonyl-, Decyl-, *iso*-Decyl-, 2-Propylheptyl-, Undecyl- und Tridecyl- Resten.

In einer Ausführungsform ist für den Fall, dass das erfindungsgemäße Gemisch epoxidierte Fettsäureester von *iso*-Nonanol enthält,

- der Anteil an Derivaten des Alkoholatrestes von *n*-Nonanol in dem Gemisch umfassend epoxidierte *iso*-Nonyl-Fettsäureester kleiner als 2 Mol-%, basierend auf der Gesamtheit aller epoxidierten *iso*-Nonyl-Fettsäureester und/oder
- der Anteil des *iso*-Butens, welches bei der Synthese der Vorstufe des - bei der Herstellung der *iso*-Nonyl-Fettsäureester eingesetzten - iso-Nonanols umgesetzt wurde, basierend auf der Gesamtheit des zur Herstellung des *iso*-Nonanols eingesetzten Butens, größer als 0,5 Mol-%.

Ein bevorzugter Gegenstand der vorliegenden Erfindung ist ein Gemisch umfassend epoxidierte Fettsäureester,

- in welchem der Massenanteil an Fettsäureketten-haltigen Verbindungen welche in der Fettsäurekette keine funktionellen Gruppen einschließlich Mehrfachbindungen aufweisen, im Vergleich zu dem Gemisch der Fettsäureketten-haltigen Verbindungen, aus denen das Gemisch umfassend epoxidierte Fettsäureester hergestellt wurde, um mindestens 20 %, vorzugsweise um mindestens 40 % mit Vorzug dazu um mindestens 60 % und insbesondere um mindestens 80 % verringert ist und
- in welchem die Alkoholatreste der Fettsäureester ausgewählt sind aus Alkoholatresten, welche 1 bis 20, vorzugsweise 2 bis 15 und insbesondere 4 bis 11 Kohlenstoffatome enthalten und (neben der Alkoholatfunktion) keine weiteren funktionellen Gruppen einschließlich Mehrfachbindungen aufweisen, wobei in dem Fall, dass das Gemisch umfassend epoxidierte Fettsäureester epoxidierte Fettsäureester von *iso*-Nonanol enthält, der Anteil an Derivaten des Alkoholatrestes von *n*-Nonanol in dem Gemisch umfassend epoxidierte *iso*-Nonyl-Fettsäureester kleiner als 2 Mol-%, basierend auf der Gesamtheit aller epoxidierten *iso*-Nonyl-Fettsäureester, ist und/oder der Anteil des *iso*-Butens, welches bei der Synthese der Vorstufe des - bei der Herstellung der *iso*-Nonyl-Fettsäureester eingesetzten - iso-Nonanols umgesetzt wurde, basierend auf der Gesamtheit des zur Herstellung des *iso*-Nonanols eingesetzten Butens, größer als 0,5 Mol-% ist.

In einer Ausführungsform enthält das erfindungsgemäße Gemisch umfassend epoxidierte Fettsäureester weniger als 90 Massen-%, weniger als 75 Massen-%, weniger als 50 Massen-% oder keine epoxidierten Fettsäureester, welche *iso*-Nonanolreste enthalten, basierend auf der Gesamtheit aller epoxidierten Fettsäureester.

Bevorzugt weisen die Alkoholatreste von mindestens 50 Massen-%, bevorzugt mindestens 65 Massen-%, besonders bevorzugt mindestens 85 Massen-% und insbesondere mindestens 95 Massen-% aller Fettsäureester in dem erfindungsgemäßen Gemisch umfassend epoxidierte Fettsäureester dieselbe Anzahl an Kohlenstoffatomen auf.

In einer anderen Ausführungsform sind in einem erfindungsgemäßen Gemisch zwei, drei, vier oder mehr epoxidierte Fettsäureester enthalten, welche sich in den Alkoholatresten der epoxidierten Fettsäureester unterscheiden. Beispielsweise kann ein erfindungsgemäßes Gemisch 20 bis 80 Massen-% epoxidierte Fettsäureester mit 5 Kohlenstoffatomen im Alkoholatrest und 20 bis 80 Massen-% epoxidierte Fettsäureester mit 9 Kohlenstoffatomen im Alkoholatrest enthalten, wobei die Anteile wiederum auf die Gesamtheit aller Fettsäurekettenhaltigen Komponenten bezogen sind.

Mit Vorzug enthält das erfindungsgemäße Gemisch umfassend epoxidierte Fettsäureester weniger als 20 Massen-%, vorzugsweise weniger als 15 Massen-%, mit besonderem Vorzug weniger als 10 Massen-% und insbesondere weniger als 5 Massen-% an Verbindungen, welche keine Fettsäureester sind.

Bevorzugt enthält das erfindungsgemäße Gemisch umfassend epoxidierte Fettsäureester weniger als 10 Massen-%, bevorzugt weniger als 8 Massen-%, besonders bevorzugt weniger als 6 Massen-% und insbesondere weniger als 4 Massen-% an nicht-epoxidierten Fettsäureketten-haltigen Verbindungen, welche in der Fettsäurekette keine funktionellen Gruppen einschließlich Mehrfachbindungen aufweisen, bezogen auf die Gesamtheit aller epoxidierten und nicht-epoxidierten Fettsäureketten-haltigen Verbindungen.

Ein bevorzugter Gegenstand der vorliegenden Erfindung ist ein Gemisch umfassend epoxidierte Fettsäureester,

- in welchem der Massenanteil an Fettsäureketten-haltigen Verbindungen welche in der Fettsäurekette keine funktionellen Gruppen einschließlich Mehrfachbindungen aufweisen, im Vergleich zu dem Gemisch der Fettsäureketten-haltigen Verbindungen, aus denen das Gemisch umfassend epoxidierte Fettsäureester hergestellt wurde, um mindestens 20 %, vorzugsweise um mindestens 40 % mit Vorzug dazu um mindestens 60 % und insbesondere um mindestens 80 % verringert ist,

- in welchem die Alkoholatreste der Fettsäureester ausgewählt sind aus Alkoholatresten, welche 1 bis 20, vorzugsweise 2 bis 15 und insbesondere 4 bis 11 Kohlenstoffatome enthalten und (neben der Alkoholatfunktion) keine weiteren funktionellen Gruppen einschließlich Mehrfachbindungen aufweisen, wobei in dem Fall, dass das Gemisch umfassend epoxidierte Fettsäureester epoxidierte Fettsäureester von *iso*-Nonanol enthält, der Anteil an Derivaten des Alkoholatrestes von *n*-Nonanol in dem Gemisch umfassend epoxidierte *iso*-Nonyl-Fettsäureester kleiner als 2 Mol-%, basierend auf der Gesamtheit aller epoxidierten *iso*-Nonyl-Fettsäureester, ist und/oder der Anteil des *iso*-Butens, welches bei der Synthese der Vorstufe des - bei der Herstellung der *iso*-Nonyl-Fettsäureester eingesetzten - iso-Nonanols umgesetzt wurde, basierend auf der Gesamtheit des zur Herstellung des *iso*-Nonanols eingesetzten Butens, größer als 0,5 Mol-% ist, und
- in dem vorzugsweise weniger als 20 Massen-%, vorzugsweise weniger als 15 Massen-%, mit besonderem Vorzug weniger als 10 Massen-% und insbesondere weniger als 5 Massen-% an Verbindungen enthalten sind, welche keine Fettsäureester sind.

Gegenstand der vorliegenden Erfindung ist zudem ein Verfahren zur Herstellung eines Gemisches umfassend epoxidierte Fettsäureester umfassend folgende Verfahrensschritte:

- Verringerung des Anteils der Fettsäuren beziehungsweise Fettsäureester, welche in der Fettsäurekette keine funktionellen Gruppen einschließlich Mehrfachbindungen aufweisen, vor der Epoxidierung der Fettsäure(n)/-ester ODER
- Verringerung des Anteils der Fettsäuren beziehungsweise Fettsäureester, welche in der Fettsäurekette keine funktionellen Gruppen einschließlich Mehrfachbindungen aufweisen, nach der Epoxidierung der Fettsäuren beziehungsweise Fettsäureester,
- im Falle der epoxidierten Fettsäuren eine Veresterung und
- optional im Falle der epoxidierten Fettsäureester eine Umesterung.

Gegenstand der vorliegenden Erfindung ist demnach ein Verfahren zur Herstellung eines Gemisches umfassend epoxidierte Fettsäureester umfassend folgende Verfahrensschritte:

- Verringerung des Anteils der gesättigten Fettsäuren beziehungsweise gesättigten Fettsäureester vor der Epoxidierung der Fettsäure(n)/-ester ODER
- Verringerung des Anteils der nicht-epoxidierten Fettsäuren beziehungsweise nicht-epoxidierten Fettsäureestern nach der Epoxidierung der Fettsäuren beziehungsweise Fettsäureester,
- im Falle der epoxidierten Fettsäuren eine Veresterung und
- optional im Falle der epoxidierten Fettsäureester eine Umesterung.

Mittels dieses Verfahrens und der nachfolgend beschriebenen Verfahren können die oben beschriebenen Gemische umfassend epoxidierte Fettsäureester hergestellt werden.

In einer Ausführungsform ist das erfindungsgemäße Verfahren gekennzeichnet durch folgende Schritte:

a) Gewinnung eines Fettsäuregemisches beziehungsweise Fettsäureestergemisches, vorzugsweise aus einem pflanzlichen Öl oder einem Gemisch von mindestens zwei pflanzlichen Ölen,
b) Verringerung des Anteils der Fettsäuren beziehungsweise der Fettsäureester, welche in der Fettsäurekette keine funktionellen Gruppen einschließlich Mehrfachbindungen aufweisen, im Gemisch aus Schritt a),
c) Epoxidierung des Gemisches aus Schritt b),
d) Veresterung beziehungsweise optional Umesterung des Gemisches aus Schritt c).

Bevorzugt ist ein Verfahren, gekennzeichnet durch folgende Schritte:

a) Gewinnung eines Fettsäuregemisches beziehungsweise Fettsäureestergemisches, vorzugsweise aus einem pflanzlichen Öl oder einem Gemisch von mindestens zwei pflanzlichen Ölen,
b) Verringerung des Anteils der gesättigten Fettsäuren beziehungsweise der gesättigten Fettsäureester im Gemisch aus Schritt a),
c) Epoxidierung des Gemisches aus Schritt b),
d) Veresterung beziehungsweise optional Umesterung des Gemisches aus Schritt c).

Das erfindungsgemäße Verfahren kann folgende Schritte umfassen:

a) Gewinnung eines Fettsäuregemisches, vorzugsweise aus einem pflanzlichen Öl oder einem Gemisch von mindestens zwei pflanzlichen Ölen,

b) Verringerung des Anteils der gesättigten Fettsäuren im Fettsäuregemisch aus Schritt a), vorzugsweise mittels Kristallisation,

c) Epoxidierung des Fettsäuregemisches aus Schritt b),

d) Veresterung des Gemisches umfassend epoxidierte Fettsäuren aus Schritt c).

Alternativ kann das erfindungsgemäße Verfahren folgende Schritte umfassen:

a) Gewinnung eines Fettsäureestergemisches, vorzugsweise aus einem pflanzlichen Öl oder einem Gemisch von mindestens zwei pflanzlichen Ölen,

b) Verringerung des Anteils der gesättigten Fettsäureester im Fettsäureestergemisch aus Schritt a), vorzugsweise mittels Kristallisation,

c) Epoxidierung des Fettsäureestergemisches aus Schritt b),

d) optional Umesterung des Gemisches umfassend epoxidierte Fettsäureester aus Schritt c).

Bei diesem Verfahren kann zusätzlich zwischen den Schritten b) und c) eine Umesterung erfolgen.

Die Verringerung des Anteils gesättigter Fettsäuren beziehungsweise gesättigter Fettsäureester wird vorzugsweise mittels Kristallisation, insbesondere mittels Kristallisation nach Vermischen der Fettsäuren beziehungsweise Fettsäureester mit Harnstoff und Alkohol, beispielsweise Ethanol oder Methanol, durchgeführt.

In einer anderen Ausführungsform umfasst das erfindungsgemäße Verfahren folgende Schritte:

a) Gewinnung eines Fettsäuregemisches beziehungsweise Fettsäureestergemisches, vorzugsweise aus einem pflanzlichen Öl oder einem Gemisch von mindestens zwei pflanzlichen Ölen,

b) Epoxidierung des Gemisches aus Schritt a),

c) Verringerung des Anteils der Fettsäuren beziehungsweise der Fettsäureester, welche in der Fettsäurekette keine funktionellen Gruppen einschließlich Mehrfachbindungen aufweisen, im Gemisch aus Schritt b),

d) Veresterung beziehungsweise optional Umesterung des Gemisches aus Schritt c).

Bevorzugt ist ein Verfahren umfassend folgende Schritte:

a) Gewinnung eines Fettsäuregemisches beziehungsweise Fettsäureestergemisches, vorzugsweise aus einem pflanzlichen Öl oder einem Gemisch von mindestens zwei pflanzlichen Ölen,

b) Epoxidierung des Gemisches aus Schritt a),

c) Verringerung des Anteils der nicht-epoxidierten Fettsäuren beziehungsweise der nicht-epoxidierten Fettsäureester im Gemisch aus Schritt b),

d) Veresterung beziehungsweise optional Umesterung des Gemisches aus Schritt c).

Das erfindungsgemäße Verfahren kann folgende Schritte umfassen:

a) Gewinnung eines Fettsäureestergemisches, vorzugsweise aus einem pflanzlichen Öl oder einem Gemisch von mindestens zwei pflanzlichen Ölen,

b) Epoxidierung des Fettsäureestergemisches aus Schritt a),

c) Verringerung des Anteils der nicht-epoxidierten Fettsäureester im Gemisch umfassend epoxidierte Fettsäureester aus Schritt b), vorzugsweise mittels Destillation,

d) optional Umesterung des Gemisches umfassend epoxidierte Fettsäureester aus Schritt c).

Alternativ kann das erfindungsgemäße Verfahren folgende Schritte umfassen:

a) Gewinnung eines Fettsäuregemisches, vorzugsweise aus einem pflanzlichen Öl oder einem Gemisch von mindestens zwei pflanzlichen Ölen,

b) Epoxidierung des Fettsäuregemisches aus Schritt a),

c) Verringerung des Anteils der nicht-epoxidierten Fettsäuren im Gemisch umfassend epoxidierte Fettsäuren aus Schritt b), vorzugsweise mittels Destillation,

d) Veresterung des Gemisches umfassend epoxidierte Fettsäuren aus Schritt c).

In dem erfinderischen Verfahren können zusätzliche Schritte wie Aufreinigung oder die Vereinigung zweier Ansätze für einen gemeinsamen nachfolgenden Verfahrensschritt oder die Aufteilung eines Ansatzes für getrennte nachfolgende Verfahrensschritte zwischen dem Schritt a) und dem Schritt b), zwischen dem Schritt b) und dem Schritt c) und/oder zwischen dem Schritt c) und dem Schritt d) durchgeführt werden.

Vorzugsweise wird in dem erfinderischen Verfahren der Massenanteil der Fettsäurekettenhaltigen Verbindungen, welche in der Fettsäurekette keine funktionellen Gruppen einschließlich Mehrfachbindungen aufweisen, im Vergleich zu dem Gemisch der Fettsäureketten-haltigen Verbindungen, aus denen das Gemisch umfassend epoxidierte Fettsäureester hergestellt wurde, um mindestens 20 %, vorzugsweise um mindestens 40 % mit Vorzug dazu um mindestens 60 % und insbesondere um mindestens 80 % verringert.

Ein bevorzugter Gegenstand der vorliegenden Erfindung ist ein Verfahren zur Herstellung eines Gemisches umfassend epoxidierte Fettsäureester,

- in welchem der Massenanteil der Fettsäureketten-haltigen Verbindungen, welche in der Fettsäurekette keine funktionellen Gruppen einschließlich Mehrfachbindungen aufweisen, im Vergleich zu dem Gemisch der Fettsäureketten-haltigen Verbindungen, aus denen das Gemisch umfassend epoxidierte Fettsäureester hergestellt wurde, um mindestens 20 %, vorzugsweise um mindestens 40 % mit Vorzug dazu um mindestens 60 % und insbesondere um mindestens 80 % verringert wird,
- in welchem die Alkoholatreste der Fettsäureester ausgewählt sind aus Alkoholatresten, welche 1 bis 20, vorzugsweise 2 bis 15 und insbesondere 4 bis 11 Kohlenstoffatome enthalten und (neben der Alkoholatfunktion) keine weiteren funktionellen Gruppen einschließlich Mehrfachbindungen aufweisen, wobei in dem Fall, dass das Gemisch umfassend epoxidierte Fettsäureester epoxidierte Fettsäureester von *iso*-Nonanol enthält, der Anteil an Derivaten des Alkoholatrestes von *n*-Nonanol in dem Gemisch umfassend epoxidierte *iso*-Nonyl-Fettsäureester kleiner als 2 Mol-%, basierend auf der Gesamtheit aller epoxidierten *iso*-Nonyl-Fettsäureester, ist und/oder der Anteil des *iso*-Butens, welches bei der Synthese der Vorstufe des - bei der Herstellung der *iso*-Nonyl-Fettsäureester eingesetzten - iso-Nonanols umgesetzt wurde, basierend auf der Gesamtheit des zur Herstellung des *iso*-Nonanols eingesetzten Butens, größer als 0,5 Mol-% ist, und
- in welchem vorzugsweise ein Gemisch hergestellt wird, in dem weniger als 20 Massen-%, vorzugsweise weniger als 15 Massen-%, mit besonderem Vorzug weniger als 10 Massen-% und insbesondere weniger als 5 Massen-% an Verbindungen enthalten sind, welche keine Fettsäureester sind,
- umfassend folgende Verfahrensschritte:
- Verringerung des Anteils der Fettsäuren beziehungsweise Fettsäureester, welche in der Fettsäurekette keine funktionellen Gruppen einschließlich Mehrfachbindungen aufweisen, vor der Epoxidierung der Fettsäure(n)/-ester ODER
- Verringerung des Anteils der Fettsäuren beziehungsweise Fettsäureestern, welche in der Fettsäurekette keine funktionellen Gruppen einschließlich Mehrfachbindungen aufweisen, nach der Epoxidierung der Fettsäuren beziehungsweise Fettsäureester,
- im Falle der epoxidierten Fettsäuren eine Veresterung und
- optional im Falle der epoxidierten Fettsäureester eine Umesterung.

Bevorzugt ist ein Verfahren zur Herstellung eines Gemisches umfassend epoxidierte Fettsäureester,

- in welchem der Massenanteil der Fettsäureketten-haltigen Verbindungen, welche in der Fettsäurekette keine funktionellen Gruppen einschließlich Mehrfachbindungen aufweisen, im Vergleich zu dem Gemisch der Fettsäureketten-haltigen Verbindungen, aus denen das Gemisch umfassend epoxidierte Fettsäureester hergestellt wurde, um mindestens 20 %, vorzugsweise um mindestens 40 % mit Vorzug dazu um mindestens 60 % und insbesondere um mindestens 80 % verringert wird,
- in welchem die Alkoholatreste der Fettsäureester ausgewählt sind aus Alkoholatresten, welche 1 bis 20, vorzugsweise 2 bis 15 und insbesondere 4 bis 11 Kohlenstoffatome enthalten und (neben der Alkoholatfunktion) keine weiteren funktionellen Gruppen einschließlich Mehrfachbindungen aufweisen, wobei in dem Fall, dass das Gemisch umfassend epoxidierte Fettsäureester epoxidierte Fettsäureester von *iso*-Nonanol enthält, der Anteil an Derivaten des Alkoholatrestes von *n*-Nonanol in dem Gemisch umfassend epoxidierte *iso*-Nonyl-Fettsäureester kleiner als 2 Mol-%, basierend auf der Gesamtheit aller epoxidierten *iso*-Nonyl-Fettsäureester, ist und/oder der Anteil des *iso*-Butens, welches bei der Synthese der Vorstufe des - bei der Herstellung der *iso*-Nonyl-Fettsäureester eingesetzten - iso-Nonanols umgesetzt wurde, basierend auf der Gesamtheit des zur Herstellung des *iso*-Nonanols eingesetzten Butens, größer als 0,5 Mol-% ist, und
- in welchem vorzugsweise ein Gemisch hergestellt wird, in dem weniger als 20 Massen-%, vorzugsweise weniger als 15 Massen-%, mit besonderem Vorzug weniger als 10 Massen-% und insbesondere weniger als 5 Massen-% an Verbindungen enthalten sind, welche keine Fettsäureester sind,
- umfassend folgende Schritte:

    a) Gewinnung eines Fettsäureestergemisches, vorzugsweise aus einem pflanzlichen Öl oder einem Gemisch von mindestens zwei pflanzlichen Ölen,

b) Epoxidierung des Fettsäureestergemisches aus Schritt a),

c) Verringerung des Anteils der nicht-epoxidierten Fettsäureester im Gemisch umfassend epoxidierte Fettsäureester aus Schritt b), vorzugsweise mittels Destillation,

d) optional Umesterung des Gemisches umfassend epoxidierte Fettsäureester aus Schritt c).

Gegenstand der vorliegenden Erfindung ist zudem ein Gemisch umfassend epoxidierte Fettsäureester, welches gemäß einem der beschriebenen Verfahren hergestellt wurde.

Gegenstand der vorliegenden Erfindung ist ein Gemisch umfassend epoxidierte Fettsäureester hergestellt durch

- Verringerung des Anteils der Fettsäuren beziehungsweise Fettsäureester, welche in der Fettsäurekette keine funktionellen Gruppen einschließlich Mehrfachbindungen aufweisen, vor der Epoxidierung der Fettsäuren beziehungsweise Fettsäureester ODER
- Verringerung des Anteils der Fettsäuren beziehungsweise Fettsäureester, welche in der Fettsäurekette keine funktionellen Gruppen einschließlich Mehrfachbindungen aufweisen, nach der Epoxidierung der Fettsäuren beziehungsweise Fettsäureester,
- im Falle der epoxidierten Fettsäuren eine Veresterung und
- optional im Falle der epoxidierten Fettsäureester eine Umesterung.

Bevorzugt ist ein Gemisch umfassend epoxidierte Fettsäureester hergestellt durch

- Verringerung des Anteils der gesättigten Fettsäuren beziehungsweise gesättigten Fettsäureester vor der Epoxidierung der Fettsäuren beziehungsweise Fettsäureester ODER
- Verringerung des Anteils der nicht-epoxidierten Fettsäuren beziehungsweise nicht-epoxidierten Fettsäureester nach der Epoxidierung der Fettsäuren beziehungsweise Fettsäureester,
- im Falle der epoxidierten Fettsäuren eine Veresterung und
- optional im Falle der epoxidierten Fettsäureester eine Umesterung.

Vorzugsweise wurde das erfindungsgemäße Gemisch hergestellt durch

a) Gewinnung eines Fettsäuregemisches beziehungsweise Fettsäureestergemisches, vorzugsweise aus einem pflanzlichen Öl oder einem Gemisch von mindestens zwei pflanzlichen Ölen,

b) Verringerung des Anteils der gesättigten Fettsäuren beziehungsweise der gesättigten Fettsäureester im Gemisch aus Schritt a), vorzugsweise mittels Kristallisation, beispielsweise nach Zusatz von Harnstoff und Alkohol,

c) Epoxidierung des Gemisches aus Schritt b),

d) Veresterung beziehungsweise optional Umesterung des Gemisches aus Schritt c).

Wird in diesem Verfahren im Schritt a) ein Fettsäureestergemisch eingesetzt, so findet optional zwischen den Schritten b) und c) eine Umesterung statt.

Ebenfalls bevorzugt ist die Herstellung des erfindungsgemäßen Gemisches durch

a) Gewinnung eines Fettsäuregemisches beziehungsweise Fettsäureestergemisches, vorzugsweise aus einem pflanzlichen Öl oder einem Gemisch von mindestens zwei pflanzlichen Ölen,

b) Epoxidierung des Gemisches aus Schritt a),

c) Verringerung des Anteils der nicht-epoxidierten Fettsäuren beziehungsweise der nicht-epoxidierten Fettsäureester im Gemisch aus Schritt b), vorzugsweise mittels Destillation,

d) Veresterung beziehungsweise optional Umesterung des Gemisches aus Schritt c).

Ein bevorzugter Gegenstand der vorliegenden Erfindung ist ein Gemisch umfassend epoxidierte Fettsäureester

- in welchem der Massenanteil an Fettsäureketten-haltigen Verbindungen welche in der Fettsäurekette keine funktionellen Gruppen einschließlich Mehrfachbindungen aufweisen, im Vergleich zu dem Gemisch der Fettsäureketten-haltigen Verbindungen, aus denen das Gemisch umfassend epoxidierte Fettsäureester hergestellt wurde, um mindestens 20 %, vorzugsweise um mindestens 40 % mit Vorzug dazu um mindestens 60 % und insbesondere um mindestens 80 % verringert ist,
- in welchem die Alkoholatreste der Fettsäureester ausgewählt sind aus Alkoholatresten, welche 1 bis 20, vorzugsweise 2 bis 15 und insbesondere 4 bis 11 Kohlenstoffatome enthalten und (neben der Alkoholatfunktion) keine weiteren funktionellen Gruppen einschließlich Mehrfachbindungen aufweisen, wobei in dem Fall, dass das Gemisch umfassend epoxidierte Fettsäureester epoxidierte Fettsäureester von *iso*-Nonanol enthält, der Anteil an Derivaten des Alkoholatrestes von *n*-Nonanol in dem Gemisch umfassend epoxidierte *iso*-Nonyl-Fettsäureester kleiner als 2 Mol-%, basierend auf der Gesamtheit aller epoxidierten *iso*-Nonyl-Fettsäureester, ist und/oder der Anteil des *iso*-Butens, welches bei der Synthese der Vorstufe des - bei der Herstellung der *iso*-Nonyl-Fettsäureester eingesetzten - iso-Nonanols umgesetzt wurde, basierend auf der Gesamtheit des zur Herstellung des *iso*-Nonanols eingesetzten Butens, größer als 0,5 Mol-% ist,

- in dem vorzugsweise weniger als 20 Massen-%, vorzugsweise weniger als 15 Massen-%, mit besonderem Vorzug weniger als 10 Massen-% und insbesondere weniger als 5 Massen-% an Verbindungen enthalten sind, welche keine Fettsäureester sind
- und welches hergestellt wurde durch
- Verringerung des Anteils der Fettsäuren beziehungsweise Fettsäureester, welche in der Fettsäurekette keine funktionellen Gruppen einschließlich Mehrfachbindungen aufweisen, vor der Epoxidierung der Fettsäuren beziehungsweise Fettsäureester ODER
- Verringerung des Anteils der Fettsäuren beziehungsweise Fettsäureester, welche in der Fettsäurekette keine funktionellen Gruppen einschließlich Mehrfachbindungen aufweisen, nach der Epoxidierung der Fettsäuren beziehungsweise Fettsäureester,
- im Falle der epoxidierten Fettsäuren eine Veresterung und
- optional im Falle der epoxidierten Fettsäureester eine Umesterung.

Bevorzugt ist ein Gemisch umfassend epoxidierte Fettsäureester

- in welchem der Massenanteil an Fettsäureketten-haltigen Verbindungen welche in der Fettsäurekette keine funktionellen Gruppen einschließlich Mehrfachbindungen aufweisen, im Vergleich zu dem Gemisch der Fettsäureketten-haltigen Verbindungen, aus denen das Gemisch umfassend epoxidierte Fettsäureester hergestellt wurde, um mindestens 20 %, vorzugsweise um mindestens 40 % mit Vorzug dazu um mindestens 60 % und insbesondere um mindestens 80 % verringert ist,
- in welchem die Alkoholatreste der Fettsäureester ausgewählt sind aus Alkoholatresten, welche 1 bis 20, vorzugsweise 2 bis 15 und insbesondere 4 bis 11 Kohlenstoffatome enthalten und (neben der Alkoholatfunktion) keine weiteren funktionellen Gruppen einschließlich Mehrfachbindungen aufweisen, wobei in dem Fall, dass das Gemisch umfassend epoxidierte Fettsäureester epoxidierte Fettsäureester von *iso*-Nonanol enthält, der Anteil an Derivaten des Alkoholatrestes von *n*-Nonanol in dem Gemisch umfassend epoxidierte *iso*-Nonyl-Fettsäureester kleiner als 2 Mol-%, basierend auf der Gesamtheit aller epoxidierten *iso*-Nonyl-Fettsäureester, ist und/oder der Anteil des *iso*-Butens, welches bei der Synthese der Vorstufe des - bei der Herstellung der *iso*-Nonyl-Fettsäureester eingesetzten - iso-Nonanols umgesetzt wurde, basierend auf der Gesamtheit des zur Herstellung des *iso*-Nonanols eingesetzten Butens, größer als 0,5 Mol-% ist,
- in dem vorzugsweise weniger als 20 Massen-%, vorzugsweise weniger als 15 Massen-%, mit besonderem Vorzug weniger als 10 Massen-% und insbesondere weniger als 5 Massen-% an Verbindungen enthalten sind, welche keine Fettsäureester sind
- und welches hergestellt wurde durch

a) Gewinnung eines Fettsäureestergemisches, vorzugsweise aus einem pflanzlichen Öl oder einem Gemisch von mindestens zwei pflanzlichen Ölen,
b) Epoxidierung des Fettsäureestergemisches aus Schritt a),
c) Verringerung des Anteils der nicht-epoxidierten Fettsäureester im Gemisch umfassend epoxidierte Fettsäureester aus Schritt b), vorzugsweise mittels Destillation,
d) optional Umesterung des Gemisches umfassend epoxidierte Fettsäureester aus Schritt c).

Gegenstand der vorliegenden Erfindung ist zudem die Verwendung eines erfindungsgemäßen Gemisches als Weichmacher für Polymere.

Geeignete Polymere sind vorzugsweise ausgewählt aus der Gruppe, die gebildet wird durch Polyvinylchlorid (PVC), Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatome(n), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymeren, insbesondere Polyvinylidenfluorid (PVDF), Polytetrafluorethylen (PTFE), Polyvinylacetat (PVAc), Polyvinylalkohol (PVA), Polyvinylacetale, insbesondere Polyvinylbutyral (PVB), Polystyrolpolymere, insbesondere Polystyrol (PS), expandierbares Polystyrol (EPS), Acrylnitril-Styrol-Acrylat (ASA), Styrolacrylnitril (SAN), Acrylnitril-Butadien-

Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi und Silikone.

Bevorzugte Polymere sind Polyvinylchlorid, Co-Polymere von Vinylchlorid mit Vinylacetat oder mit Butylacrylat, Polyalkylmethacrylat (PAMA), Polyvinylbutyral (PVB), Polyurethan, Polysulfide, Polymilchsäure (PLA), Polyhydroxybutyral (PHB) und Nitrocellulose.

Weiterer Gegenstand der vorliegenden Erfindung ist ein Mittel enthaltend ein erfindungsgemäßes Gemisch sowie ein oder mehrere Polymere aus der Gruppe die gebildet wird von Polyvinylchlorid, Co-Polymere von Vinylchlorid mit Vinylacetat oder mit Butylacrylat, Polyalkylmethacrylat (PAMA), Polyvinylbutyral (PVB), Polyurethan, Polysulfide, Polymilchsäure (PLA), Polyhydroxybutyral (PHB) und Nitrocellulose.

Bezogen auf 100 Massenteile Polymer enthalten bevorzugte Mittel vorzugsweise von 5 bis 200, bevorzugt von 10 bis 150 Massenteile an Weichmacher.

Bevorzugt ist die Verwendung des erfindungsgemäßen Gemisches als Weichmacher für Polyvinylchlorid und dementsprechend sind Mittel, welche das erfindungsgemäße Gemisch und PVC enthalten, besonders bevorzugt.

Bevorzugt ist das Polymer als Suspensions-, Masse-, Mikrosuspensions- oder Emulsions-PVC.

Bevorzugte erfindungsgemäße Mittel enthalten neben dem erfindungsgemäßen Gemisch mindestens einen weiteren Weichmacher. Eine besonders bevorzugte Ausführungsform des erfindungsgemäßen Mittels liegt vor, wenn dieses weniger als 5 Massen-% Phthalat-haltige Verbindungen enthält und insbesondere Phthalat-frei ist. Die weiteren Weichmacher werden vorzugsweise ausgewählt aus der Gruppe der Adipate, Benzoate, chlorierten Kohlenwasserstoffe, Citrate, Cyclohexandicarboxylate, epoxidierten Fettsäureestern, epoxidierte Pflanzenöle, epoxidierte acetylierte Glyceride, Furandicarboxylate, Phosphate, Phthalate (vorzugsweise in möglichst geringen Mengen), Succinate, Sulfonamide, Sulfonate, Terephthalate, Trimellitate oder oligomeren oder polymeren Ester auf Basis von Adipin-, Bernstein- oder Sebacinsäure. Besonders bevorzugt sind Alkylbenzoate, Dialkyladipate, Glycerinester, Citronensäuretrialkylester, acylierte Citronensäuretrialkylester, Trialkyltrimellitate, Glykoldibenzoate, Dialkylterephthalate, Ester der Furandicarbonsäure, Dialkanoylester von Dianhydrohexitolen (z. B. Isosorbit) und Dialkylester der 1,2-, 1,3- oder 1,4-Cyclohexandicarbonsäure.

In einer Ausführungsform enthält das erfindungsgemäße Mittel neben dem erfindungsgemäßen Gemisch umfassend epoxidierte Fettsäureester weniger als 20 Massen-%, weniger als 10 Massen-% oder keinen weiteren Weichmacher, wobei die Massen-% auf der Gesamtmasse des Mittels basieren.

Erfindungsgemäße Mittel enthalten vorzugsweise neben dem Polymer oder einer Mischung mehrerer Polymere und dem erfindungsgemäßen Gemisch ein oder mehrere Additive aus der Gruppe der Thermostabilisatoren, Füllstoffe, Pigmente, Treibmittel, Biozide, UV-Stabilisatoren, Co-Stabilisatoren, Antioxidantien, Viskositätsregler, Gleitmittel und Färbemittel.

Gegenstand der vorliegenden Erfindung ist zudem die Verwendung des erfindungsgemäßen Gemisches zur Verbesserung der Gelierung eines Plastisols und/oder zur Verringerung der Viskosität eines Plastisols und/oder zur Verringerung der Glasübergangstemperatur eines mit dem erfindungsgemäßen Gemisch hergestellten Mittels und/oder zur Verbesserung der Thermostabilität eines mit dem erfindungsgemäßen Gemisch hergestellten Mittels.

Die erfindungsgemäßen Gemische beziehungsweise die erfindungsgemäßen Mittel können in Klebstoffen, Dichtungsmassen, Beschichtungsmassen, Lacken, Farben, Plastisolen, Schäumen, Kunstleder, Fußbodenbelägen (z. B. Deckschicht), Dachbahnen, Unterbodenschutz, Gewebebeschichtungen, Kabeln oder Drahtisolierungen, Schläuchen, Extrusionsartikeln, sowie in Folien, insbesondere für den Automobilinnenbereich und auch in Tapeten oder Tinten eingesetzt werden.

**Experimenteller** Teil:

Im experimentellen Teil werden die Begriffe "gesättigt", "abgereichert" und "nicht-abgereichert" wie in der Beschreibung definiert gebraucht.

Beispiel 1:

Herstellung des epoxidierten Fettsäuremethylesters aus Leinöl durch Epoxidierung von Leinölmethylester

Der Fettsäuremethylester (588 g, ca. 2 mol, Mosselmann, Methyl esters of linseed oil) wird in eine Epoxidierungsapparatur (Doppelmantelreaktor mit integrierter Kühlschlange, Rührer, Tauchrohr, Tropftrichter, Dosierpumpe, Thermometer, pH-Meter und aufgesetztem Rückflusskühler) vorgelegt. Die Apparatur wird anschließend für 60 Minuten mit 6 L $N_2$/ Stunde über das Tauchrohr gespült. Während der Reaktion wird Stickstoff über den Reaktorinhalt geleitet, um eine Inertisierung der Gasphase sicher zu stellen. Das Startmaterial wird langsam unter Rühren auf 45 °C aufgeheizt. Nach Erreichen der Zieltemperatur wird der pH-Wert mittels Zugabe von Natronlauge (20 Massen-% in Wasser) auf pH 4,5 eingestellt. Insgesamt werden im Laufe der Reaktion 690 mL Natronlauge hinzugegeben. Anschließend wird Peressigsäure (35 %, PERACLEAN 35, Evonik Industries, 3,96 mol) mittels Dosierpumpe über 2 Stunden zugegeben. Über die komplette Zeit wird der pH-Wert durch Zutropfen von Natronlauge konstant gehalten. Die Reaktionstemperatur wird über die gesamte Reaktionszeit auf 45 °C gehalten (+/- 1,5 °C). Nach Start der Reaktion wird die Reaktionswärme über die Kühlschlange abgeführt (Kühlung in Intervallen). Nach vollständiger Zugabe erfolgt eine Nachreaktion über 22 Stunden.

Der Reaktionsaustrag wird in einen Scheidetrichter gefüllt und 30 Minuten bei Raumtemperatur absitzen gelassen. Die wässrige Phase wird abgelassen und verworfen. Die organische Phase wird in einen 2 Liter Reaktionskolben gefüllt und mit Tauchrohr, Rührer und Thermometer sowie mit einer Claisenbrücke mit Vakuumanschluss verbunden. Der Reaktionsaustrag wird drei Mal mit 25 % Wasser, bezogen auf die Einwaage, gewaschen. Anschließend wird bei 60 °C unter maximalem Vakuum 15 Minuten getrocknet und auf 160 °C aufgeheizt. Nach Erreichen der Temperatur wird der Kolbeninhalt mit Stickstoff gestrippt. Dazu wird die Stickstoffmenge so eingestellt, dass der Druck von maximalem Vakuum auf 40 mbar ansteigt. Nach zwei Stunden wird die Heizung abgeschaltet und unter Einleiten von Stickstoff auf 90 °C abgekühlt. Der Ester wird über einen Büchnertrichter mit Filterpapier und vorgepresstem Filterkuchen aus Filterhilfsmittel (Perlite Typ D14) mittels Vakuum in eine Saugflasche gefiltert.

Herstellung der "abgereicherten" Gemische, NMR und GC/MS

Beispiel 2:

Herstellung eines "abgereicherten" Gemisches umfassend epoxidierte Fettsäuremethylester durch Verringerung des Anteils der nicht-epoxidierten Fettsäuremethylester in einem Gemisch umfassend epoxidierte Fettsäuremethylester.

(Beispiel 2-S: aus Sojaöl; Beispiel 2-L: aus Leinöl)

5000 g des jeweiligen epoxidierten Fettsäuremethylesters werden in einem Kurzwegverdampfer vom Typ KDL 5 (Fa. UIC GmbH; Verdampfer, Destillat- und Rückflussstrom können separat über Thermostaten beheizt werden) unter nachfolgend aufgeführten Bedingungen destilliert.

Tabelle 1: experimentelle Daten Beispiel 2-S und Beispiel 2-L

| | epox. Fettsäuremethylester aus Sojaöl (Beispiel 2-S) | epox. Fettsäuremethylester aus Leinöl (Beispiel 2-L) |
|---|---|---|
| Druck | < $10^{-3}$ mbar | < $10^{-3}$ mbar |
| Temperatur im Verdampfer | 120 °C | 116 °C |
| Temperatur Destillat | 40 °C | 27 °C |
| Temperatur Rückstand | 40 °C | 40 °C |
| Wischerdrehzahl | 313 rpm | 305 rpm |
| Drehzahl Einförderpumpe | 400 rpm | 300 rpm |
| Rückstand (= "abgereichertes" Gemisch) | 74 Massen-% | 81 Massen-% |
| Destillat | 26 Massen-% | 19 Massen-% |
| epox. Fettsäuremethylester aus Sojaöl: Reflex100 (Fa. PolyOne) epox. Fettsäuremethylester aus Leinöl: aus Beispiel 1 | | |

Beispiel 3:

Bestimmung der Zusammensetzung von Gemischen umfassend epoxidierte Fettsäuremethylester mittels "on column" Hochtemperatur-GC/MS mit FID

Analysenparameter:

| Gerät: | Agilent GC 7890 / Agilent MSD 5975 |
|---|---|
| Kapillarsäule: | 30 m DB-5HT; 0,32 mm Innendurchmesser; 0,1 $\mu$m Film |
| Ionisation: | Elektronenstoßionisation, 70 eV und chemische Ionisation mit Ammoniak als Reaktant-Gas |
| Trägergas: | Helium |
| Säulenfluss: | 2,6 mL / min |
| Ofentemperatur: | 80 °C - 10 °C / min - 400 °C (20 min) |
| Detektor (FID): | 400 °C |
| Injektor: | cool-on-column, 80 °C - 140 °C / min - 400 °C |
| Injektionsvolumen: | 0,5 $\mu$L |
| Probenvorbereitung: | 100 mg der Probe und 20 mg *n*-Hexadecan zusammen einwiegen und 40,0 mL *n*-Heptan zugeben |

Identifizierung der Fettsäuremethylester

Die Identifizierung der Fettsäuremethylester erfolgte durch Vergleich der Retentionszeiten in den Probelösungen und einer Standardlösung mit bekannten Fettsäuremethylestern in Kombination mit einer Strukturaufklärung mittels GC/MS-Analyse.

Quantifizierung der Fettsäuremethylester

Die quantitative Auswertung der Fettsäuremethylester wurde gegen *n*-Hexadecan als internen Standard unter Berücksichtigung von theoretisch berechneten Korrekturfaktoren durchgeführt:

Gehalt in Massen-% = (Peakfläche Fettsäuremethylester x berechneter Korrekturfaktor x Einwaage interner Standard x 100 %) / (Peakfläche interner Standard x Einwaage Probe)

Berechnete Korrekturfaktoren:

| | |
|---|---|
| Hexadecansäuremethylester | 1,21 |
| Octadecansäuremethylester | 1,19 |
| Eicosansäuremethylester | 1,17 |
| Epoxy-octadecansäuremethylester | 1,40 |
| Diepoxy-octadecansäuremethylester | 1,47 |
| Triepoxy-octadecansäuremethylester | 1,63 |

Tabelle 2: Zusammensetzung der Gemische umfassend epoxidierte Fettsäuremethylester aus Sojaöl (gemäß GC/MS), Angaben in Massen-% aller Fettsäureketten-haltiger Verbindungen

| | "nicht-abgereichertes" Gemisch-S (Edukt aus Beispiel 2-S) | "abgereichertes" Gemisch-S (Rückstand aus Beispiel 2-S) |
|---|---|---|
| **Hexadecansäuremethylester** | **9,53** | **0,33** |
| **Octadecansäuremethylester** | **3,71** | **1,06** |
| Epoxy-octadecansäuremethylester | 23,1 | 20,0 |
| **Eicosansäuremethylester** | **0,35** | **0,26** |
| Diepoxy-octadecansäuremethylester | 34,2 | 41,6 |
| Diepoxy-octadecansäuremethylester | 19,7 | 24,9 |
| **Docosansäuremethylester** | **0,32** | **0,38** |
| Triepoxy-octadecansäuremethylester | 2,68 | 3,73 |
| Triepoxy-octadecansäuremethylester | 1,76 | 2,47 |
| Triepoxy-octadecansäuremethylester | 1,72 | 2,41 |
| Triepoxy-octadecansäuremethylester | 0,79 | 1,12 |
| **Summe "gesättigte" Ester** | **13,91** | **2,03** |

In dem Gemisch umfassend epoxidierte Fettsäuremethylester basierend auf Sojaöl (erfindungsgemäßes "abgereichertes" Gemisch-S) wurde der Massenanteil der Fettsäurekettenhaltigen Verbindungen, welche in der Fettsäurekette keine funktionellen Gruppen einschließlich Mehrfachbindungen aufweisen, im Vergleich zu dem Gemisch der Fettsäureketten-haltigen Verbindungen, aus denen das Gemisch umfassend epoxidierte Fettsäureester hergestellt wurde ("nicht-abgereichertes" Gemisch, Edukt aus Beispiel 2-S), um 85,4 % verringert.

Tabelle 3: Zusammensetzung der Gemische umfassend epoxidierte Fettsäuremethylester aus Leinöl (gemäß GC/MS), Angaben in Massen-% aller Fettsäureketten-haltiger Verbindungen

| | "nicht-abgereichertes" Gemisch-L (Edukt aus Beispiel 2-L) | "abgereichertes" Gemisch-L (Rückstand aus Beispiel 2-L) |
|---|---|---|
| **Hexadecansäuremethylester** | **4,30** | **0,20** |
| **Octadecansäuremethylester** | **3,76** | **0,79** |
| Epoxy-octadecansäuremethylester | 21,9 | 15,8 |
| **Eicosansäuremethylester** | **0,13** | **0,08** |
| Diepoxy-octadecansäuremethylester | 11,2 | 11,8 |
| Diepoxy-octadecansäuremethylester | 4,53 | 5,11 |
| Diepoxy-octadecansäuremethylester | 8,50 | 9,17 |
| Triepoxy-octadecansäuremethylester | 2,62 | 3,02 |
| Triepoxy-octadecansäuremethylester | 17,1 | 20,4 |
| Triepoxy-octadecansäuremethylester | 10,6 | 12,1 |
| Triepoxy-octadecansäuremethylester | 8,26 | 9,12 |
| Triepoxy-octadecansäuremethylester | 4,53 | 5,25 |
| **Summe "gesättigte" Ester** | **8,19** | **1,07** |

In dem Gemisch umfassend epoxidierte Fettsäuremethylester basierend auf Leinöl (erfindungsgemäßes "abgereichertes" Gemisch-L) wurde der Massenanteil der Fettsäurekettenhaltigen Verbindungen, welche in der Fettsäurekette keine funktionellen Gruppen einschließlich Mehrfachbindungen aufweisen, im Vergleich zu dem Gemisch der Fettsäureketten-haltigen Verbindungen, aus denen das Gemisch umfassend epoxidierte Fettsäureester hergestellt wurde ("nicht-abgereichertes" Gemisch, Edukt aus Beispiel 2-L) um 86,9 % verringert.

Anhand der Massenspektren kann keine Aussage über die Position der Epoxidgruppen in der Fettsäurekette getroffen werden. Bei mehrfach epoxidierten Fettsäureestern treten Regio- und Diastereomere auf, was die Mehrfachnennung einzelner Verbindungen in den Tabellen 2 und 3 erklärt.

Beispiel 4:

Bestimmung des Doppelbindungsanteils und der Anzahl der Epoxidgruppen pro Fettsäurekette mittels NMR in Gemischen umfassend epoxidierte Fettsäuremethylester

Die Bestimmung des Anteils von Doppelbindungen und Epoxidgruppen erfolgt durch $^1$H-NMR-Spektroskopie. Für die Aufnahme der Spektren werden zum Beispiel 50 mg Substanz in 0,6 mL CDCl$_3$ (enthaltend 1 Massen-% TMS) gelöst und in ein NMR-Röhrchen mit einem Durchmesser von 5 mm gefüllt.

Die NMR-spektroskopischen Untersuchungen können prinzipiell mit jedem handelsüblichen NMR-Gerät durchgeführt werden. Für die vorliegenden NMR-spektroskopischen Untersuchungen wurde ein Gerät vom Typ Avance 500 der Firma Bruker eingesetzt. Die Spektren wurden bei einer Temperatur von 303 K mit einem Delay (Verzögerung) von d1 = 5 Sekunden, 32 Scans (Durchgänge), einer Pulslänge von ca. 9,5 $\mu$s und einer Sweep Width (Spektrale Breite) von 10000 Hz mit einem 5 mm BBO-Probenkopf (broad band observer; Breitbandbeobachtung) aufgenommen. Die Resonanzsignale werden gegen die chemische Verschiebung von Tetramethylsilan (TMS = 0 ppm) als internen Standard aufgezeichnet. Mit anderen handelsüblichen NMR-Geräten werden mit den gleichen Betriebsparametern vergleichbare Ergebnisse erhalten.

Zur Bestimmung der Anteile der einzelnen Strukturelemente sind zunächst die zugehörigen Signale im NMR-Spektrum zu identifizieren. Nachfolgend sind die verwendeten Signale mit ihrer Lage im Spektrum und der Zuordnung zu entsprechenden Strukturelementen aufgelistet:

- Die Signale im Bereich 4,8 bis 6,4 ppm wurden den $^1$H-Kernen der Doppelbindungen zugeordnet.
- Die Signale im Bereich 3,25 bis 2,85 ppm wurden den $^1$H-Kernen der Epoxide zugeordnet.

Zur Quantifizierung der Anteile werden Referenzsignale bekannter Größe benötigt. Folgende Signale wurden verwendet:

- Die Signale der Methylengruppe in Nachbarschaft zur Carboxylgruppe der Fettsäure, welche als enges Signalmultiplett um 2,3 ppm im Spektrum zur Resonanz kommen.
- Die Signale der Methylengruppe in Nachbarschaft zum Sauerstoff der veresterten Alkohols entsprechend dem Strukturelement -CH$_2$-O-, welche im Bereich 3,9 bis 4,2 ppm im Spektrum zur Resonanz kommen.

Die Quantifizierung erfolgt durch Bestimmung der Fläche unter den jeweiligen Resonanzsignalen, d. h. der vom Signal von der Grundlinie eingeschlossenen Fläche. Handelsübliche NMR-Geräte verfügen über Vorrichtungen zur Integration der Signalfläche. In den vorliegenden NMR-spektroskopischen Untersuchung wurde die Integration mit Hilfe der Software "TOPSPIN", Version 3.1 durchgeführt.

Zur Berechnung des Anteils der Doppelbindungen wird der Integralwert x der Doppelbindungssignale im Bereich 4,8 bis 6,4 ppm durch den Integralwert der Referenzmethylengruppe r geteilt.

Zur Berechnung des Anteils der Epoxide wird der Integralwert y der Epoxidsignale im Bereich 2,85 bis 3,25 ppm durch den Integralwert der Referenzmethylengruppe r geteilt.

Es werden die relativen Anteile der Strukturelemente Doppelbindung und Epoxidgruppe je Fettsäurerest/Fettsäurekette enthalten.

Tabelle 4: Doppelbindungsanteile und Anzahl der Epoxidgruppen pro Fettsäurekette

| | "nicht-abgereichertes" Gemisch-S (Edukt aus Beispiel 2-S) | "abgereichertes" Gemisch-S (Rückstand aus Beispiel 2-S) | "nicht-abgereichertes" Gemisch-L (Edukt aus Beispiel 2-L) | "abgereichertes" Gemisch-L (Rückstand aus Beispiel 2-L) |
|---|---|---|---|---|
| Doppelbindungs-anteil | 0,01 | 0,01 | 0,13 | 0,14 |
| Anzahl EpoxidGruppen | 1,49 | 1,76 | 1,85 | 2,11 |

Beispiele 5 bis 9:

Herstellung der erfindungsgemäßen Gemische durch Umesterung des "abgereicherten" Gemisches-S und des "abgereicherten" Gemisches-L (Rückstand aus Beispiel 2-S und Beispiel 2-L)

Der Rückstand aus Beispiel 2-S ($m_{Ester}$) beziehungsweise aus Beispiel 2-L ($m_{Ester}$) wird mit dem jeweiligen Alkohol ($m_{Alkohol}$) n-Butanol, n-Pentanol, 2-Ethylhexanol, iso-Nonanol beziehungsweise 2-Propylheptanol in einer Umesterungsapparatur mit einem Reaktionskolben, Rührer, Tauchrohr, Thermometer, Destillationskopf, 20 cm Raschigringkolonne, Vakuumteiler und Auffangkolben vorgelegt, eine Stunde mit 6 L $N_2$/ Stunde über das Tauchrohr gespült und auf 45 °C aufgeheizt. Nach Erreichen der 45 °C wird Kaliummethanolat (KOMe, (Evonik Industries, 32 % in Methanol, $m_{Kat}$) als Katalysator zugegeben und zunächst leichtes Vakuum angelegt. Von der Kopftemperatur abhängig wird das Vakuum soweit wie möglich maximiert, ohne dass ein höherwertiger Alkohol zusammen mit dem Methanol abdestilliert wird. Der aufgefangene Alkohol wird gewogen (Auffangkolben und Kühlfalle, $m_{Destillat}$) Am Ende der Reaktion lag ein Vakuum von $p_{Vakuum}$ an. Der Reaktionsverlauf wird mittels GC Analyse verfolgt. Dazu wird jede Stunde eine Probe genommen, der Katalysator mit Essigsäure zersetzt und die Probe über einen Spritzenfilter filtriert. Die Reaktion ist beendet, wenn der Methylestergehalt < 2 Flächen-% beträgt ($t_{Reaktion}$). Liegt der Methylestergehalt nach $t_{Nachdosierung}$ noch über 2 Flächen-% wurden nochmals $m_{Alkohol-Nachdosierung}$ der Alkoholkomponente und $m_{Kat-Nachdosierung}$ KOMe nachdosiert. Nach Ende der Reaktion wird der Katalysator durch Zugabe von $m_{HOAc}$ Essigsäure (HOAc) zersetzt, wonach jeweils die organische Phase von der wässrigen getrennt wird.

Der Reaktionsaustrag aus der Umesterung wird in einen Reaktionskolben, bestückt mit Claisenbrücke inklusive Vakuumteiler, Tauchrohr mit Stickstoffanschluss sowie Thermometer, umgefüllt und mit 2 % Aktivkohle, bezogen auf die Masse an Reaktionsaustrag, versetzt. Der Ansatz wird unter Rühren mit Stickstoff gespült. Unter maximalem Vakuum (<1 mbar) wird langsam aufgeheizt und die Temperatur entsprechend des Destillationsanfalls langsam bis auf $T_{Destillation}$ erhöht. An Leichtsiedern wird eine Masse von $m_{Leichtsieder}$ abgetrennt und dann verworfen. Die Reaktionsmasse wurde auf $T_{Filtration}$ abgekühlt und dann filtriert. Dazu wurde der Ester über einen Büchnertrichter mit Filterpapier und vorgepresstem Filterkuchen aus Filterhilfsmittel (Perlite Typ D14) mittels Vakuum in eine Saugflasche gefiltert.

Beispiel 5:

Umesterung des - an nicht-epoxidierten Fettsäuremethylestern abgereicherten - Gemisches umfassend epoxidierte Fettsäuremethylester zu den Butylestern (Beispiel 5-S: aus Sojaöl; Beispiel 5-L: aus Leinöl)

Alkohol = Butanol (Sigma Aldrich, >99%)

Tabelle 5: Herstellung der Butylester des "abgereicherten" Gemisches-S und des "abgereicherten" Gemisches-L

| | Butylester "abgereichertes" Gemisch-S | Butylester "abgereichertes" Gemisch-L |
|---|---|---|
| Ausgangsmaterial | Rückstand aus Beispiel 2-S | Rückstand aus Beispiel 2-L |
| $m_{Ester}$ | 685 g | 708 g |
| $M_{Alkohol}$ | 233,5 g | 233,5 g |

(fortgesetzt)

| | Butylester "abgereichertes" Gemisch-S | Butylester "abgereichertes" Gemisch-L |
|---|---|---|
| Ausgangsmaterial | Rückstand aus Beispiel 2-S | Rückstand aus Beispiel 2-L |
| $m_{Kat}$ | 59 g | 59 g |
| $m_{Destillat}$ | 290,5 g | 290,5 g |
| $m_{Alkohol-Nachdosierung}$ | 100 g | 100 g |
| $m_{Kat-Nachdosierung}$ | 59 g | 59 g |
| $M_{HOAc}$ | 47,2 g | 47,2 g |
| $m_{Leichtsieder}$ | 14,6 g | 14,8 g |
| $T_{Destillation}$ | 160 °C | 160 °C |
| $T_{Filtration}$ | <80°C | <80°C |
| $t_{Nachdosierung}$ | 9,5 h | 9,5 h |
| $t_{Reaktion}$ | 17,5 h | 17,5 h |
| $p_{Vakuum}$ | 2 mbar | 1 mbar |

Beispiel 6:

Umesterung des - an nicht-epoxidierten Fettsäuremethylestern abgereicherten - Gemisches umfassend epoxidierte Fettsäuremethylester zu den Pentylestern (Beispiel 6-S: aus Sojaöl; Beispiel 6-L: aus Leinöl)

Alkohol = *n*-Pentanol (Sigma Aldrich, >99%)

Tabelle 6: Herstellung der Pentylester des "abgereicherten" Gemisches-S und des "abgereicherten" Gemisches-L

| | *n*-Pentylester "abgereichertes" Gemisch-S | *n*-Pentylester "abgereichertes" Gemisch-L |
|---|---|---|
| Ausgangsmaterial | Rückstand aus Beispiel 2-S | Rückstand aus Beispiel 2-L |
| $m_{Ester}$ | 685 g | 351 g |
| $M_{Alkohol}$ | 278 g | 139 g |
| $m_{Kat}$ | 59 g | 29,5 g |
| $m_{Destillat}$ | 233 g | 114 g |
| $m_{Alkohol-Nachdosierung}$ | 191 g | 114 g |
| $m_{Kat-Nachdosierung}$ | 0 | 0 |
| $M_{HOAc}$ | 23,6 g | 11,8 g |
| $m_{Leichtsieder}$ | 87 g | 20 g |
| $T_{Destillation}$ | 160 °C | 160 °C |
| $T_{Filtration}$ | < 80 °C | < 80 °C |
| $t_{Nachdosierung}$ | 1 h, 2 h, 3 h | 1 h, 2 h, 3 h, 4 h |
| $t_{Reaktion}$ | 4 h | 1,5 h |
| $p_{Vakuum}$ | < 1 mbar | < 1 mbar |

Beispiel 7:

Umesterung des - an nicht-epoxidierten Fettsäuremethylestern abgereicherten - Gemisches umfassend epoxidierte Fettsäuremethylester zu den 2-Ethylhexylestern (Beispiel 7-S: aus Sojaöl; Beispiel 7-L: aus Leinöl)

Alkohol = 2-Ethylhexanol (Sigma Aldrich, >99%)

Tabelle 7: Herstellung der 2-Ethylhexylester des "abgereicherten" Gemisches-S und des "abgereicherten" Gemisches-L

|  | 2-Ethylhexylester "abgereichertes" Gemisch-S | 2-Ethylhexylester "abgereichertes" Gemisch-L |
|---|---|---|
| Ausgangsmaterial | Rückstand aus Beispiel 2-S | Rückstand aus Beispiel 2-L |
| $m_{Ester}$ | 685 g | 701 g |
| $M_{Alkohol}$ | 410 g | 410 g |
| $m_{Kat}$ | 59 g | 59 g |
| $m_{Destillat}$ | 104 g | 97 g |
| $m_{Alkohol-Nachdosierung}$ | 0 | 97 g |
| $m_{Kat-Nachdosierung}$ | 0 | 0 |
| $M_{HOAc}$ | 23,6 g | 23,6 g |
| $m_{Leichtsieder}$ | 158 g | 282 g |
| $T_{Destillation}$ | 160 °C | 160 °C |
| $T_{Filtration}$ | < 80 °C | < 80 °C |
| $t_{Nachdosierung}$ | keine Nachdosierung | 1 h, 2,5 h |
| $t_{Reaktion}$ | 4 h | 4,5 h |
| $p_{Vakuum}$ | 3 mbar | 6 mbar |

Beispiel 8:

Umesterung des - an nicht-epoxidierten Fettsäuremethylestern abgereicherten - Gemisches umfassend epoxidierte Fettsäuremethylester zu den *iso*-Nonylestern (Beispiel 8-S: aus Sojaöl; Beispiel 8-L: aus Leinöl)

Alkohol = *iso*-Nonanol (Evonik Industries, >99%)

Tabelle 8: Herstellung der *iso*-Nonylester des "abgereicherten" Gemisches-S und des "abgereicherten" Gemisches-L

|  | *iso*-Nonylester "abgereichertes" Gemisch-S | *iso*-Nonylester "abgereichertes" Gemisch-L |
|---|---|---|
| Ausgangsmaterial | Rückstand aus Beispiel 2-S | Rückstand aus Beispiel 2-L |
| $m_{Ester}$ | 685 g | 701 g |
| $M_{Alkohol}$ | 454 g | 454 g |
| $m_{Kat}$ | 59 g | 59 g |
| $m_{Destillat}$ | 108 g | 113 g |
| $m_{Alkohol-Nachdosierung}$ | 106 g | 113 g |
| $m_{Kat-Nachdosierung}$ | 0 | 0 |

(fortgesetzt)

| | iso-Nonylester "abgereichertes" Gemisch-S | iso-Nonylester "abgereichertes" Gemisch-L |
|---|---|---|
| Ausgangsmaterial | Rückstand aus Beispiel 2-S | Rückstand aus Beispiel 2-L |
| $M_{HOAc}$ | 23,6 g | 23,6 g |
| $m_{Leichtsieder}$ | 256 g | 241 g |
| $T_{Destillation}$ | 160 °C | 160 °C |
| $T_{Filtration}$ | < 80 °C | < 80 °C |
| $t_{Nachdosierung}$ | 1 h, 2 h | 1 h, 2 h |
| $t_{Reaktion}$ | 5,75 h | 7 h |
| $p_{Vakuum}$ | 2 mbar | < 1 mbar |

Beispiel 9:

Umesterung des - an nicht-epoxidierten Fettsäuremethylestern abgereicherten - Gemisches umfassend epoxidierte Fettsäuremethylester zu den 2-Propylheptylestern (Beispiel 9-S: aus Sojaöl; Beispiel 9-L: aus Leinöl)

Alkohol = 2-Propylheptanol (Evonik Industries, >99%)

Tabelle 9: Herstellung der 2-Propylheptylester des "abgereicherten" Gemisches-S und des "abgereicherten" Gemisches-L

| | 2-Propylheptylester "abgereichertes" Gemisch-S | 2-Propylheptylester "abgereichertes" Gemisch-L |
|---|---|---|
| Ausgangsmaterial | Rückstand aus Beispiel 2-S | Rückstand aus Beispiel 2-L |
| $m_{Ester}$ | 685 g | 701 g |
| $m_{Alkohol}$ | 454 g | 498 g |
| $m_{Kat}$ | 59 g | 59 g |
| $m_{Destillat}$ | 108 g | 142 g |
| $m_{Alkohol-Nachdosierung}$ | 108 g | 0 |
| $m_{Kat-Nachdosierung}$ | 0 | 0 |
| $m_{HOAc}$ | 23,6 g | 23,6 g |
| $m_{Leichtsieder}$ | 235 g | 34 g |
| $T_{Destillation}$ | 160 °C | 160 °C |
| $T_{Filtration}$ | 80 °C | 80 °C |
| $t_{Nachdosierung}$ | 1 h, 2 h | keine Nachdosierung |
| $t_{Reaktion}$ | 3,25 h | 7 h |
| $p_{Vakuum}$ | 2 mbar | 6 mbar |

Beispiele 10 bis 13:

Herstellung der "nicht-abgereicherten" Gemische (Vergleichssubstanzen) durch Umesterung der epoxidierten, "nicht-abgereicherten" Fettsäuremethylester

Die "nicht-abgereicherten" Gemische werden analog zu dem in den Beispielen 5 bis 9 beschriebenen Verfahren durch Umesterung hergestellt, wobei statt des Rückstands aus Beispiel 2-S beziehungsweise aus Beispiel 2-L der jeweilige epoxidierte Fettsäuremethylester aus Sojaöl (Edukt aus Beispiel 2-S) beziehungsweise aus Leinöl (Edukt aus Beispiel 2-L) eingesetzt wird.

epox. Fettsäuremethylester aus Sojaöl: Edukt aus Beispiel 2-S, Reflex100 (Fa. PolyOne)

epox. Fettsäuremethylester aus Leinöl: aus Beispiel 1, Edukt aus Beispiel 2-L

Beispiel 10:

Umesterung des "nicht-abgereicherten" Gemisches zu den Butylestern (Beispiel 10-S: aus Sojaöl; Beispiel 10-L: aus Leinöl)

Alkohol = Butanol (Sigma Aldrich, >99%)

Tabelle 10: Herstellung der Butylester des "nicht-abgereicherten" Gemisches-S und des "nicht-abgereicherten" Gemisches-L

|  | Butylester "nicht-abgereichertes" Gemisch-S | Butylester "nicht-abgereichertes" Gemisch-L |
|---|---|---|
| Ausgangsmaterial | Edukt aus Beispiel 2-S | Edukt aus Beispiel 2-L |
| $m_{Ester}$ | 685 g | 685 g |
| $M_{Alkohol}$ | 233,5 g | 233,5 g |
| $m_{Kat}$ | 59 g | 59 g |
| $m_{Destillat}$ | 158 g | 90 g |
| $m_{Alkohol-Nachdosierung}$ | 0 | 0 |
| $m_{Kat-Nachdosierung}$ | 0 | 0 |
| $M_{HOAc}$ | 23,6 g | 23,6 g |
| $m_{Leichtsieder}$ | 14,3 g | 14,3 g |
| $T_{Destillation}$ | 160 °C | 160 °C |
| $T_{Filtration}$ | < 80 °C | < 80 °C |
| $t_{Nachdosierung}$ | keine Nachdosierung | keine Nachdosierung |
| $t_{Reaktion}$ | 9,5 h | 8 h |
| $p_{Vakuum}$ | < 1 mbar | 26 mbar |

Beispiel 11:

Umesterung des "nicht-abgereicherten" Gemisches zu den 2-Ethylhexylestern (Beispiel 11-S: aus Sojaöl; Beispiel 11-L: aus Leinöl)

Alkohol = 2-Ethylhexanol (Sigma Aldrich, >99%)

Tabelle 11: Herstellung der 2-Ethylhexylester des "nicht-abgereicherten" Gemisches-L

|  | 2-Ethylhexanolester "nicht-abgereichertes" Gemisch-L |
|---|---|
| Ausgangsmaterial | Edukt aus Beispiel 2-L |
| $m_{Ester}$ | 996 g |
| $M_{Alkohol}$ | 396 g |
| $m_{Kat}$ | 84 g |
| $m_{Destillat}$ | 100 g |

(fortgesetzt)

| | 2-Ethylhexanolester "nicht-abgereichertes" Gemisch-L |
|---|---|
| Ausgangsmaterial | Edukt aus Beispiel 2-L |
| $m_{Alkohol-Nachdosierung}$ | 100 g |
| $m_{Kat-Nachdosierung}$ | 0 |
| $M_{HOAc}$ | 34 g |
| $m_{Leichtsieder}$ | 219 g |
| $T_{Destillation}$ | 160 °C |
| $T_{Filtration}$ | <80°C |
| $t_{Nachdosierung}$ | 1 h, 2 h |
| $t_{Reaktion}$ | 4 h |
| $p_{Vakuum}$ | 8 mbar |

Beispiel 12:

Umesterung des "nicht-abgereicherten" Gemisches zu den *iso*-Nonylestern (Beispiel 12-S: aus Sojaöl; Beispiel 12-L: aus Leinöl)

Alkohol = *iso*-Nonanol (Evonik Industries, >99%)

Tabelle 12: Herstellung der *iso*-Nonylester des "nicht-abgereicherten" Gemisches-S und des "nicht-abgereicherten" Gemisches-L

| | *iso*-Nonylester "nicht-abgereichertes" Gemisch-S | *iso*-Nonylester "nicht-abgereichertes" Gemisch-L |
|---|---|---|
| Ausgangsmaterial | Edukt aus Beispiel 2-S | Edukt aus Beispiel 2-L |
| $m_{Ester}$ | 685 g | 685 g |
| $M_{Alkohol}$ | 454 g | 454 g |
| $m_{Kat}$ | 59 g | 59 g |
| $m_{Destillat}$ | 117 g | 102 g |
| $m_{Alkohol-Nachdosierung}$ | 0 | 0 |
| $m_{Kat-Nachdosierung}$ | 0 | 0 |
| $M_{HOAc}$ | 23,6 g | 23,6 g |
| $m_{Leichtsieder}$ | 149 g | 154 g |
| $T_{Destillation}$ | 160 °C | 160 °C |
| $T_{Filtration}$ | 80 °C | 80 °C |
| $t_{Nachdosierung}$ | keine Nachdosierung | keine Nachdosierung |
| $t_{Reaktion}$ | 6 h | 6 h |
| $p_{Vakuum}$ | 3 mbar | < 1 mbar |

Beispiel 13:

Umesterung des "nicht-abgereicherten" Gemisches zu den 2-Propylheptylestern (Beispiel 13-S: aus Sojaöl; Beispiel 13-L: aus Leinöl)

Alkohol = 2-Propylheptanol (Evonik Industries, >99%)

Tabelle 13: Herstellung der 2-Propylheptylester des "nicht-abgereicherten" Gemisches-S und des "nicht-abgereicherten" Gemisches-L

|  | 2-Propylheptylester "nicht-abgereichertes" Gemisch-S | 2-Propylheptylester "nicht-abgereichertes" Gemisch-L |
|---|---|---|
| Ausgangsmaterial | Rückstand aus Beispiel 2-S | Rückstand aus Beispiel 2-L |
| $m_{Ester}$ | 685 g | 701 g |
| $M_{Alkohol}$ | 498 g | 498 g |
| $m_{Kat}$ | 59 g | 59 g |
| $m_{Destillat}$ | 115 g | 118 g |
| $m_{Alkohol-Nachdosierung}$ | 115 g | 118 g |
| $m_{Kat-Nachdosierung}$ | 0 | 0 |
| $M_{HOAc}$ | 23,6 g | 23,6 g |
| $m_{Leichtsieder}$ | 275 g | 282 g |
| $T_{Destillation}$ | 160 °C | 160 °C |
| $T_{Filtration}$ | 80 °C | 80 °C |
| $t_{Nachdosierung}$ | 1 h, 2 h | 1 h, 2,5 h |
| $t_{Reaktion}$ | 3,25 h | 3,5 h |
| $p_{Vakuum}$ | 2 mbar | 4 mbar |

Beispiel 14:

Umesterung von epoxidiertem Sojabohnenöl (Beispiel 14-S) beziehungsweise von epoxidiertem Leinöl (Beispiel 14-L) zu den *n*-Pentylestern

In einer Umesterungsapparatur mit einem Reaktionskolben, Rührer, Tauchrohr, Thermometer, Destillationskopf, 20 cm Raschigringkolonne, Vakuumteiler und Auffangkolben wird Kaliummethanolat (Evonik Industries AG, 32 % in Methanol, $m_{Kat}$) mit der Hälfte des *n*-Pentanols ($m_{Alkohol}$/2) vermischt. Unter maximalem Vakuum ($p_{Vakuum}$) wird auf 45 °C aufgeheizt. Das anfallende Destillat wird, ohne Rückfluss, vollständig abgenommen ($m_{Destillat}$). Die zweite Hälfte des Alkohols wird anschließend, nach Fluten des Reaktors mit Stickstoff, zügig bei konstanter Sumpftemperatur zugetropft. Nach einer Stunde werden Auffangkolben und Kühlfalle ausgewogen, und das angefallene Destillat wird durch zusätzliches *n*-Pentanol ersetzt ($m_{Alkohol-Nachdosierung}$). Dieser Vorgang wird nach der zweiten Stunde Reaktionszeit wiederholt (Wert wird in $m_{Alkohol-Nachdosierung}$ zusammengefasst). Es wird davon ausgegangen, dass nach zwei Stunden Reaktionszeit das Kaliummethanolat vollständig zum entsprechenden Kaliumalkoholat umgesetzt wurde. In den Kolben mit Katalysator und Alkohol wird bei 45 °C epoxidiertes Sojabohnenöl beziehungsweise epoxidiertes Leinöl ($m_{Ester}$) zügig zugetropft, woraufhin die Sumpftemperatur konstant gehalten wird. Der Reaktionsverlauf wird mittels HT-GC Analyse verfolgt. Dazu wird jede Stunde eine Probe genommen, der Katalysator mit Essigsäure zersetzt und die Probe über einen Spritzenfilter gefiltert. Zur Bestimmung der Mono-, Di- und Triglyceride mittels HT-GC muss die Probe silyliert werden. Sobald der verbleibende Rest an Mono-, Di- und Triglyceriden kleiner als 2 Flächen-% ist ($t_{Reaktion}$), wird die Reaktion durch Zugabe von Essigsäure ($m_{HOAC}$) abgebrochen.

Der Kolbeninhalt wird in einen Scheidetrichter gegeben und absitzen lassen. Das Glycerin (untere Phase) wird abgelassen. Die obere Phase wird zwei Mal bei 80 °C unter Stickstoffeinperlung mit Wasser gewaschen (30 % Wasser bezogen auf den Kolbeninhalt).

Von der organischen Phase wird eine Säurezahl bestimmt, und anschließend mit 1,1-facher stöchiometrischer Menge (zu der bestimmten Säurezahl) an 10 %-iger Natronlauge neutralisiert. Das Produkt wird danach erneut bei 80°C unter Stickstoffeinperlung mit Wasser gewaschen, bis das Waschwasser neutral ist (pH 7 - 8).

Der organische Phase wird in einen Reaktionskolben, bestückt mit Claisenbrücke inklusive Vakuumteiler, Tauchrohr mit Stickstoffanschluss sowie Thermometer, umgefüllt und mit 2 % Aktivkohle bezogen auf die Masse an Reaktionsaustrag versetzt. Der Ansatz wird unter Rühren mit Stickstoff gespült. Unter maximalem Vakuum (< 1 mbar) wird langsam aufgeheizt und die Temperatur entsprechend des Destillationsanfalls langsam bis auf $T_{Destillation}$ erhöht. An Leichtsiedern wird eine Masse von $m_{Leichtsieder}$ abgetrennt und dann verworfen. Die Reaktionsmasse wurde auf $T_{Filtration}$ abgekühlt und dann filtriert. Dazu wurde der Ester über einen Büchnertrichter mit Filterpapier und vorgepresstem Filterkuchen aus Filterhilfsmittel (Perlite Typ D14) mittels Vakuum in eine Saugflasche gefiltert.

Tabelle 14: Herstellung der $n$-Pentylester des "nicht-abgereicherten" Gemisches-S und des "nicht-abgereicherten" Gemisches-L

| | $n$-Pentylester "nicht-abgereichertes" Gemisch-S | $n$-Pentylester "nicht-abgereichertes" Gemisch-L |
|---|---|---|
| Ausgangsmaterial | epoxidiertes Sojabohnenöl | epoxidiertes Leinöl |
| $m_{Ester}$ | 996 g | 996 g |
| $M_{Alkohol}$ | 396 g | 396 g |
| $m_{Kat}$ | 84 g | 84 g |
| $m_{Destillat}$ | 92 g | 100 g |
| $m_{Alkohol-Nachdosierung}$ | 235 g | 100 g |
| $m_{Kat-Nachdosierung}$ | 0 | 0 |
| $M_{HOAc}$ | 34 g | 34 g |
| $m_{Leichtsieder}$ | 92 g | 219 g |
| $T_{Destillation}$ | 160 °C | 160 °C |
| $T_{Filtration}$ | < 80 °C | < 80 °C |
| $t_{Nachdosierung}$ | 1 h, 2 h | 1 h, 1 h |
| $t_{Reaktion}$ | 4 h | 4 h |
| $p_{Vakuum}$ | 11 mbar | 8 mbar |
| epoxidiertes Sojabohnenöl: Epoxol D65, Avokal GmbH<br>epoxidiertes Leinöl: Merginat ELO, Hobum Chemicals | | |

Nachfolgend wird mittels Kurzwegverdampfer vom Typ KDL 5 (Fa. UIC GmbH; Verdampfer, Destillat- und Rückflussstrom können separat über Thermostaten beheizt werden) unter nachfolgend aufgeführten Bedingungen destilliert.

Tabelle 15: Bedingungen Destillation bei der Herstellung der $n$-Pentylester des "nicht-abgereicherten" Gemisches-S und des "nicht-abgereicherten" Gemisches-L

| | $n$-Pentylester "nicht-abgereichertes" Gemisch-S | $n$-Pentylester "nicht-abgereichertes" Gemisch-L |
|---|---|---|
| Druck | < $10^{-3}$ mbar | < $10^{-3}$ mbar |
| Temperatur im Verdampfer | 180 °C | 180 °C |
| Temperatur Destillat | 25 °C | 25 °C |
| Temperatur Rückstand | 80 °C | 80 °C |
| Wischerdrehzahl | 300 rpm | 300 rpm |
| Drehzahl Einförderpumpe | 400 rpm | 400 rpm |

(fortgesetzt)

|  | *n*-Pentylester "nicht-abgereichertes" Gemisch-S | *n*-Pentylester "nicht-abgereichertes" Gemisch-L |
|---|---|---|
| Rückstand | 12 Massen-% | 17 Massen-% |
| Destillat | 88 Massen-% | 83 Massen-% |

Vergleich der Eigenschaften von "abgereicherten" und "nicht-abgereicherten" Gemischen

Es zeigte sich, dass die "abgereicherten" Gemische durchgehend eine bessere Verträglichkeit mit PVC aufweisen als "nicht-abgereicherte" Gemische, welche sich von den "abgereicherten" Gemischen lediglich im Anteil der Fettsäureketten-haltigen Verbindungen, welche im Rahmen des vorliegenden Textes als "gesättigt" bezeichnet werden, unterscheiden.

Die Verträglichkeit der Weichmacher in den untersuchten PVC Folien wurde gemäß "Loop test" (ASTM D 3291) durchgeführt. Im Falle der "nicht-abgereicherten" Gemische epoxidierter 2-Propylheptylester von Lein- oder Sojaöl traten deutliche Ausschwitzerscheinungen auf. Deshalb ist ein Einsatz in typischen Weich-PVC Anwendungen nicht möglich. Da "nicht-abgereicherte" Gemische epoxidierter 2-Propylheptylester von Lein- oder Sojaöl nicht mit PVC verträglich sind, wurden die weiteren Eigenschaften der 2-Propylheptylestergemische bei den nachfolgenden Tests nicht bestimmt. Das erfindungsgemäße "abgereicherte" Gemisch epoxidierter 2-Propylheptylester ist hingegen mit PVC verträglich, so dass dessen Eigenschaften ebenfalls bestimmt wurden.

Es wurden in den nachfolgenden Beispielen folgende Gemische umfassend epoxidierte Fettsäureester untersucht:

Tabelle 16: untersuchte Gemische basierend auf Sojaöl

|  | "nicht-abgereichertes" Gemisch-S | "abgereichertes" Gemisch-S |
|---|---|---|
| Methylester | Reflex100 (Fa. PolyOne) | aus Beispiel 2-S |
| Butylester | aus Beispiel 10-S | aus Beispiel 5-S |
| *n*-Pentylester | aus Beispiel 14-S | aus Beispiel 6-S |
| 2-Ethylhexylester | PLS Green 8 (Petrom) | aus Beispiel 7-S |
| *iso*-Nonylester | aus Beispiel 12-S | aus Beispiel 8-S |
| 2-Propylheptylester | aus Beispiel 13-S | aus Beispiel 9-S |

Tabelle 17: untersuchte Gemische basierend auf Leinöl

|  | "nicht-abgereichertes" Gemisch-L | "abgereichertes" Gemisch-L |
|---|---|---|
| Methylester | aus Beispiel 1 | aus Beispiel 2-L |
| Butylester | aus Beispiel 10-L | aus Beispiel 5-L |
| *n*-Pentylester | aus Beispiel 14-L | aus Beispiel 6-L |
| 2-Ethylhexylester | aus Beispiel 11-L | aus Beispiel 7-L |
| *iso*-Nonylester | aus Beispiel 12-L | aus Beispiel 8-L |
| 2-Propylheptylester | aus Beispiel 13-L | aus Beispiel 9-L |

Beispiel 15:

Flüchtigkeit der "abgereicherten" und der "nicht-abgereicherten" Gemische (reine Weichmacher)

Die Flüchtigkeit von Weichmachern ist eine zentrale Eigenschaft für viele Polymeranwendungen. Hohe Flüchtigkeiten führen zu einer erhöhten Exposition der Umwelt und bei langen Lebensdauern der Produkte durch verringerte Weichmacheranteile im Polymeren zu verschlechterten mechanischen Eigenschaften. Flüchtige Weichmacher werden daher häufig nur in geringen Anteilen zu anderen Weichmachersystemen beigemischt oder überhaupt nicht eingesetzt. Besondere Bedeutung hat die Flüchtigkeit zum Beispiel in Innenraumanwendungen (Tapeten, Automobile) oder aufgrund von Richtlinien und Normen bei Kabeln oder Lebensmittelverpackungen.
Die Flüchtigkeit der reinen Weichmacher wurde mit Hilfe des Halogentrockners HB 43-S der Fa. Mettler Toledo bestimmt. Vor der Messung wurde eine leere, saubere Aluschale im Wägeteller platziert. Danach wurde die Aluschale mit einem Vlies tariert und etwa fünf Gramm Weichmacher auf das Vlies pipettiert und genau verwogen.
Durch das Schließen des Heizmoduls wurde die Messung gestartet und die Probe mit maximaler Heizrate (Voreinstellung) von Raumtemperatur auf 200 °C aufgeheizt und alle 30 Sekunden der entsprechende Masseverlust durch Verdampfung automatisch durch Wägung bestimmt. Nach 10 min wurde die Messung vom Gerät automatisch beendet.
Von jeder Probe wurde eine Doppelbestimmung durchgeführt.

Tabelle 18: Flüchtigkeit der Gemische basierend auf Sojaöl in Massen-%

|  | "nicht-abgereichertes" Gemisch-S | "abgereichertes" Gemisch-S |
|---|---|---|
| Methylester | 21,2 | 12,8 |
| Butylester | 9,6 | 5,5 |
| *n*-Pentylester | 8,8 | 4,9 |
| 2-Ethylhexylester | 5,4 | 3,3 |
| *iso*-Nonylester | 3,4 | 2,4 |
| 2-Propylheptylester | - | 2,0 |

Tabelle 19: Flüchtigkeit der Gemische basierend auf Leinöl in Massen-%

|  | "nicht-abgereichertes" Gemisch-L | "abgereichertes" Gemisch-L |
|---|---|---|
| Methylester | 16,5 | 10,3 |
| Butylester | 9,9 | 4,9 |
| *n*-Pentylester | 7,4 | 3,6 |
| 2-Ethylhexylester | 2,2 | 2,1 |
| *iso*-Nonylester | 2,2 | 1,6 |

Die erfindungsgemäßen "abgereicherten" Gemische umfassend epoxidierte Fettsäureester zeigen durchgehend eine geringere Flüchtigkeit als die "nicht-abgereicherten" Gemische.

Beispiel 16:

Herstellung von Plastisolen

Es wurden PVC-Plastisole hergestellt, wie sie beispielsweise zur Fertigung von Deckstrichfilmen für Fußbodenbeläge verwendet werden. Die Angaben in den Plastisolrezepturen sind jeweils in Massenanteilen. Die Rezepturen der Polymerzusammensetzungen sind in Tabelle 20 aufgelistet.

Tabelle 20: Plastisolrezeptur

|  | phr |
| --- | --- |
| PVC (Vestolit B 7021 - Ultra; Fa. Vestolit) | 100 |
| "abgereichertes" Gemisch beziehungsweise "nicht-abgereichertes" Gemisch | 50 |
| epoxidierten Sojabohnenöl als Co-Stabilisator (Drapex 39, Fa. Galata) | 3 |
| Thermostabilisator auf Ca/Zn-Basis (Mark CZ 149, Fa. Galata) | 2 |
| phr: parts per hundred parts resin | |

Die Weichmacher wurden vor der Zugabe auf 25 °C temperiert. Zuerst wurden die flüssigen und dann die pulverförmigen Bestandteile in einen PE-Becher eingewogen. Von Hand wurde die Mischung mit einem Salbenspatel so eingerührt, dass kein unbenetztes Pulver mehr vorhanden war. Der Mischbecher wurde dann in die Klemmvorrichtung eines Dissolverrührers eingespannt. Vor dem Eintauchen des Rührers in die Mischung wurde die Drehzahl auf 1800 Umdrehungen pro Minute eingestellt. Nach dem Einschalten des Rührers wurde so lange gerührt, bis die Temperatur an der Digitalanzeige des Thermofühlers 30,0 °C erreichte. Damit war sicher gestellt, dass die Homogenisierung des Plastisols bei einem definierten Energieeintrag erreicht wurde. Danach wurde das Plastisol sofort für weitere Untersuchungen in einem Klimaschrank auf 25,0 °C temperiert.

Beispiel 17:

Gelierverhalten von Plastisolen mit "abgereicherten" und "nicht-abgereicherten" Gemischen

Die Untersuchung des Gelierverhaltens der Plastisole wurde mit einem Physica MCR 101 im Oszillationsmodus mit einem Platte-Platte Messsystem (PP25), welches schubspannungsgesteuert betrieben wurde, vorgenommen. Eine zusätzliche Temperierhaube wurde an das Gerät angeschlossen, um eine homogene Wärmeverteilung und eine gleichmäßige Probentemperatur zu erreichen.

Folgende Parameter wurden eingestellt:

| Modus: | Temperatur-Gradient |
| --- | --- |
| Start-Temperatur: | 25 °C |
| End-Temperatur: | 180 °C |
| Heiz/Kühlrate: | 5 °C / min |
| Oszillations-Frequenz: | 4 - 0,1 Hz Rampe logarithmisch |
| Kreisfrequenz Omega: | 10 1 / s |
| Anzahl Messpunkte: | 63 |
| Messpunktdauer: | 0,5 min |
| Automatische Spaltnachführung F : | 0 N |
| Konstante Messpunktdauer | |
| Spaltweite | 0,5 mm |

Durchführung der Messung:

Auf die untere Messsystemplatte wurden mit dem Spatel einige Gramm des zu messenden Plastisols luftblasenfrei aufgetragen. Dabei wurde darauf geachtet, dass nach dem Zusammenfahren des Messsystems etwas Plastisol gleichmäßig aus dem Messsystem herausquellen konnte (nicht mehr als 6 mm rundum). Anschließend wurde die Temperierhaube über der Probe positioniert und die Messung gestartet. Bestimmt wurde die sogenannte komplexe Viskosität des Plastisols nach 24 h (Lagerung des Plastisols bei 25 °C in einem Temperierschrank der Fa. Memmert) in Abhängigkeit von der Temperatur.

Als Maß für die Gelierung wird ein deutlicher Anstieg der komplexen Viskosität betrachtet. Als Vergleichswert wird daher die Temperatur bei Erreichen einer Plastisolviskosität von 1000 Pa·s verwendet.

Tabelle 21: Gelierung der Gemische auf Basis Sojaöl nach 24 h,
Temperatur in °C bei Erreichen einer Plastisolviskosität von $10^3$ Pa·s

|  | "nicht-abgereichertes" Gemisch-S | "abgereichertes" Gemisch-S |
|---|---|---|
| Methylester | 60 | 56 |
| Butylester | 72 | 66 |
| *n*-Pentylester | 74 | 68 |
| 2-Ethylhexylester | 85 | 74 |
| *iso*-Nonylester | 88 | 78 |
| 2-Propylheptylester | - | 81 |

Tabelle 22: Gelierung der Gemische auf Basis Leinöl nach 24 h, Temperatur
in °C bei Erreichen einer Plastsolviskosität von $10^3$ Pa·s

|  | "nicht-abgereichertes" Gemisch-L | "abgereichertes" Gemisch-L |
|---|---|---|
| Methylester | 55 | 51 |
| Butylester | 63 | 61 |
| *n*-Pentylester | 66 | 65 |
| 2-Ethylhexylester | 78 | 71 |
| *iso*-Nonylester | 81 | 74 |
| 2-Propylheptylester | - | 76 |

Die Viskosität von $10^3$ Pa·s wird bei den "abgereicherten" Gemischen gleicher Alkoholkettenlänge generell bei niedrigen Temperaturen erreicht, es liegt eine bessere Gelierung vor. Diese Eigenschaft der erfindungsgemäßen, "abgereicherten" Gemische führt zu Vorteilen bei der Verarbeitbarkeit entsprechender Plastisole, da so entweder die Arbeitstemperaturen niedriger gewählt werden können (Energieersparnis) oder durch Erhöhung der Laufgeschwindigkeit der Bänder, auf denen die Plastisole gelieren, der Durchsatz erhöht werden kann.

Beispiel 18:

Effizienz der Gemische (Shore A Härte der Weich PVC Proben)

Die Shore-Härte ist ein Maß für die Weichheit eines Probekörpers. Je weiter bei einer bestimmten Messdauer eine genormte Nadel in den Probenkörper eindringen kann, desto niedriger fällt der Messwert aus. Der Weichmacher mit der höchsten Effizienz ergibt bei gleicher Weichmachermenge den niedrigsten Wert für die Shore-Härte. Da in der Praxis Formulierungen / Rezepturen häufig auf eine bestimmte Shore-Härte hin eingestellt bzw. optimiert werden, kann bei sehr effizienten Weichmachern demnach ein bestimmter Anteil in der Rezeptur eingespart werden, was eine Kostenreduktion für den Verarbeiter bedeutet.

Zur Bestimmung der Shore-Härten wurden die wie oben beschrieben hergestellten Plastisole in runde Gießformen aus Messing mit einem Durchmesser von 42 mm gegossen (Einwaage: 20,0 g). Dann wurden die Pasten in den Formen im Umlufttrockenschrank 30 min bei 200 °C geliert, nach Abkühlung entnommen und vor der Messung mindestens 24 Stunden im Klimaschrank (25 °C) gelagert. Die Dicke der Scheiben betrug ca. 12 mm.

Die Härte-Messungen wurden nach DIN 53 505 mit einem Shore-A-Messgerät der Fa. Zwick-Roell durchgeführt, der Messwert jeweils nach 3 Sekunden abgelesen. An jedem Prüfkörper wurden Messungen an drei verschiedenen Stellen durchgeführt, und der Mittelwert gebildet.

Tabelle 23: Shore A Härte von Prüfkörpern enthaltend Gemische auf Basis von Sojaöl

|  | "nicht-abgereichertes" Gemisch-S | "abgereichertes" Gemisch-S |
|---|---|---|
| Methylester | 69 | 66 |
| Butylester | 73 | 70 |
| *n*-Pentylester | 74 | 71 |
| 2-Ethylhexylester | 80 | 74 |
| *Iso*-Nonylester | 81 | 76 |
| 2-Propylheptylester | - | 78 |

Tabelle 24: Shore A Härte von Prüfkörpern enthaltend Gemische auf Basis von Leinöl

|  | "nicht-abgereichertes" Gemisch-L | "abgereichertes" Gemisch-L |
|---|---|---|
| Methylester | 64 | 64 |
| Butylester | 72 | 69 |
| *n*-Pentylester | 74 | 71 |
| 2-Ethylhexylester | 80 | 73 |
| *Iso*-Nonylester | 81 | 74 |
| 2-Propylheptylester | - | 75 |

Prüfkörper der "abgereicherten" Gemische zeigen eine geringere Shore A Härte und damit eine bessere Weichmacher-Effizienz als Prüfkörper der "nicht-abgereicherten" Gemische. Dadurch kann Weichmacher eingespart werden, was zu geringeren Rezepturkosten führt.

Beispiel 19:

Herstellung von Dryblends, Walzfellen und Pressplatten

Die für die nachfolgenden Beispiele benötigten Prüfkörper werden durch Trockenvermischen (Dryblendherstellung), Kalandrieren (Walzen) und Pressen der folgenden Rezepturen hergestellt:

Tabelle 25: Dryblend-Rezeptur

|  | phr |
|---|---|
| PVC (Solvin S271 PC; Fa. Solvay) | 100 |
| "abgereichertes" Gemisch beziehungsweise "nicht-abgereichertes" Gemisch | 67 |
| Epoxidiertes Sojabohnenöl (Drapex 39; Fa. Galata) | 3 |
| Thermostabilisator auf Ba/Zn Basis (Mark BZ 965; Fa. Galata) | 2 |

(fortgesetzt)

|  | phr |
|---|---|
| Fettsäuresalze (Mark CD 41-0137; Fa. Galata) | 0,4 |
| phr: part per hundred parts resin | |

Mit Trockenmischungen, die als Dryblends bezeichnet werden, lassen sich zum Beispiel Kabel-und Drahtisolierungen oder Fußböden und Dachbahnen herstellen.

Die Herstellung der Dryblends erfolgte in einem Brabender Planetenmischer. Ein mit vollentsalztem Wasser gefüllter Thermostat (Fa. Lauda RC6) sorgte für die Temperierung des Mischbehälters am Planetenmischer. Ein PC zeichnete die vom Mischer gesendeten Daten über ein Datenkabel in der Software "Winmix" auf.

Über die Software "Winmix" wurden folgende Parameter am Brabender Planetenmischer eingestellt.

| | |
|---|---|
| Drehzahlprogramm: | Aktiv |
| Profil: | Drehzahl 50 U/min; |
| | Haltezeit: 9 min; |
| | Anstiegszeit: 1 min |
| | Drehzahl 100 U/min; |
| | Haltezeit: 20 min |
| Knetertemperatur: | 88 °C |
| Messbereich: | 2 Nm |
| Dämpfung: | 3 |

Am Thermostat wurde eine Temperatur von 90 °C eingestellt und über eine Schlauchverbindung wurde der Mischbehälter am Brabender temperiert. Die Temperatur im Mischbehälter betrug nach einstündiger Temperierzeit 88 °C. Nachdem der Planetenmischer eine Eigenkalibrierung durchgeführt hatte, wurden die festen Bestandteile (PVC, Füllstoff, Stabilisator), welche vorher auf einer Waage (Fa. Mettler Typ XS6002S) in vierfacher Menge (vierfache Menge in g bezogen auf die Tabelle 24 in phr) in einen PE-Becher einwogen wurden, dem Mischgefäß über einen Feststofftrichter und den vorhandenen Einfüllstutzen am Brabender Mischgefäß zugeführt. Das Programm wurde gestartet und die Pulvermischung wurde 10 Minuten im Mischgefäß gerührt und temperiert, ehe die flüssigen Bestandteile, welche ebenfalls in vierfacher Menge auf der Waage in einem PE-Becher ausgewogen wurden, über einen Flüssigkeitstrichter und dem vorhandenen Einfüllstutzen am Brabender Mischgefäß, zugeführt wurden. Die Mischung wurde weitere 20 Minuten im Planetenmischer gerührt. Nach Beendigung des Programms wurde die fertige Trockenmischung (Dryblend) entnommen.

Aus diesen Dryblends werden Walzfelle hergestellt. Die Herstellung der Walzfelle erfolgte auf einem Kalander W150 AP der Fa. Collin. Der Kalander der Fa. Collin besitzt einen automatischen Probenumleger und wird über einen zusätzlichen Ölthermostat (Fa. Single Typ: STO 1-6-12-DM) temperiert. Die Steuerung erfolgt über Software der Fa. Collin.

Folgende Parameter wurden am Kalander eingestellt:

| | |
|---|---|
| Walzentemperatur [°C]: | 165 |
| Walzzeit [min]: | 5,83 |

Fünfstufiges Programm zur Herstellung des Walzfelles

| | | |
|---|---|---|
| 1. | Stufe: Plastifizierung des Dryblends | (165 °C / 60 s / 0,2 mm Spalt / 5 Upm) |
| 2. | Stufe: Änderung Walzenspalt | (165 °C / 30 s / 0,5 mm Spalt / 20 Upm) |
| 3. | Stufe: Durchmischung Schmelze | (165 °C / 170 s / 0,5 mm Spalt / 20 Upm) |

(fortgesetzt)

| | | |
|---|---|---|
| 4. | Stufe: Walzfelloptimierung | (165 °C / 30 s / 0,5 mm Spalt / 25 Upm) |
| 5. | Stufe: Walzfellabnahme | (165 °C / 60 s / 0,5 mm Spalt / 7 Upm) |

Nach dem Erreichen der Walzentemperatur wurde der Walzenspalt kalibriert. Zum Start der Messung wurde der Walzenspalt auf 0,2 mm eingestellt. Jeweils 160 Gramm eines Dryblends wurden eingewogen und bei stehenden Walzen in den Walzenspalt gegeben. Das Programm wurde gestartet. Die Walzen starteten mit einer Umdrehungszahl von 5 U/min und einer Friktion von 20 %. Nach ca. 1 min war die Plastifizierung zum größten Teil abgeschlossen und der Walzenspalt wurde auf 0,5 mm vergrößert. Es erfolgte eine 6-malige Homogenisierung mittels automatischer Umlegeeinheit am Kalander. Nach etwa 6 min wurde das Walzfell von der Walze entfernt und abgekühlt.

Die Pressplatten wurden an einer Laborpresse der Fa. Collin hergestellt. Die vorgefertigten Walzfelle (siehe oben) wurden zur Herstellung der Pressplatten verwendet. Die Seitenränder der Walzfelle wurden mit Hilfe einer Schneidemaschine entfernt, das Walzfell wurde anschließend in ca. 14,5 x 14,5 cm große Stücke geschnitten. Für 1 mm dicke Pressplatten wurden je 2 Walzfellstücke in den 15 x 15 cm großen Pressrahmen aus Edelstahl gelegt.

Folgende Parameter wurden an der Laborpresse eingestellt:

Dreiphasiges Programm:

Phase 1: Beide Platten 175 °C; Pressplattendruck: 5 bar; Phasenzeit: 60 Sekunden.
Phase 2: Beide Platten 175 °C; Pressplattendruck: 200 bar; Phasenzeit: 120 Sekunden.
Phase 3: Beide Platten 40 °C; Pressplattendruck: 200 bar; Phasenzeit: 270 Sekunden.

Die überschüssige Presslippe wurde nach Herstellung der Pressplatten entfernt.

Beispiel 20:

Massenverlust bei Aktivkohlelagerung

Aus den Pressplatten des Beispiels 19 wurden jeweils 3 Kreise von 10 $cm^2$ pro zu prüfender Rezeptur ausgestanzt. Zusätzlich wurden die Kreise mit der Schere radial angeschnitten (2 Schnitte ä 5 mm). Die Kreise wurden für eine halbe Stunde im Exsikkator (gefüllt mit KC-Trockenperlen orange) klimatisiert und anschließend ausgewogen.

Weißblechdosen (1 L, hohe Form) wurden, damit ein Druckaustausch stattfinden konnte, im Deckel mit einem Loch versehen. Der Boden der Weißblechdosen wurde mit 120 mL Aktivkohle bedeckt. Die in diesem Test eingesetzte Aktivkohle (Nr. 774408 der Fa. Roth) wurde vorher in einer Abdampfschale für 6 Stunden im Trockenschrank bei 100 +/-1 °C getrocknet und nach kurzer Abkühlung eingesetzt. Auf die Aktivkohle wurde der erste Probenkreis mittig platziert. Weitere 120 mL Aktivkohle wurden auf den Probenkreis gegeben. Insgesamt wurden die Weißblechdosen mit 480 mL Aktivkohle und 3 Probenkreisen schichtweise befüllt. Der Deckel der Weißblechdosen wurde ohne Druck auf die Dosen aufgelegt.

Die befüllten Weißblechdosen wurden bei 120 +/-1 °C für 3 Tage im Temperierschrank gelagert. Nach der Lagerung wurde die Aktivkohle von den Kreisen mittels Analysenpinsel entfernt, die Kreise zum Abkühlen 30 Minuten in einem Exsikkator gelagert und anschließend ausgewogen.

Nach der Auswaage wurden die Probenkreise wieder mit Aktivkohle zurück in die Weißblechdosen geschichtet. Hierzu wurde darauf geachtet, dass die Probenkreise wieder derselben Aktivkohle und derselben Dose zugeordnet wurden. Die Dosen wurden erneut im Temperierschrank platziert. Nach insgesamt 7 Tagen wurden die Proben dann, wie bereits beschrieben, nochmals ausgewogen.

Es wurde die prozentuale Massenveränderung von jedem Probenkreis berechnet und der Mittelwert über die 3 Kreise je Rezeptur berechnet.

Tabelle 26: Massenveränderung bei Aktivkohlelagerung in Massen-% (Gemische auf Basis Sojaöl)

|  | "nicht-abgereichertes" Gemisch-S | | "abgereichertes" Gemisch-S | |
|---|---|---|---|---|
|  | 3 Tage | 7 Tage | 3 Tage | 7 Tage |
| Methylester | -19,9 | -27,1 | -16,9 | -25,8 |
| Butylester | -13,8 | -19,7 | -8,2 | -13,1 |
| *n*-Pentylester | -12,3 | -17,4 | -6,4 | -10,8 |
| 2-Ethylhexylester | -4,8 | -8,0 | -3,5 | -6,1 |
| *iso*-Nonylester | -3,4 | -5,8 | -2,2 | -3,8 |
| 2-Propylheptylester | - | - | -2,1 | -3,7 |

Tabelle 27: Massenverlust bei Aktivkohlelagerung in Massen-% (Gemische auf Basis Leinöl)

|  | "nicht-abgereichertes" Gemisch-L | | "abgereichertes" Gemisch-L | |
|---|---|---|---|---|
|  | 3 Tage | 7 Tage | 3 Tage | 7 Tage |
| Methylester | -14,4 | -20,8 | -12,0 | -18,5 |
| Butylester | -8,9 | -13,7 | -5,1 | -9,8 |
| *n*-Pentylester | -7,4 | -11,9 | -4,2 | -8,1 |
| 2-Ethylhexylester | -3,1 | -5,4 | -2,3 | -4,2 |
| *iso*-Nonylester | -2,5 | -4,4 | -1,7 | -3,1 |
| 2-Propylheptylester | - | - | -1,5 | -2,9 |

Es konnte ein deutlich verringerter Massenverlust entsprechend einer deutlich geringeren Flüchtigkeit der "abgereicherten" Gemische festgestellt werden. Dies ist eine vorteilhafte Eigenschaft für Hochtemperaturanwendungen und Produkte mit geplant hohen Lebensdauern.

Beispiel 21:

Weichmacherwanderung / Migration nach 4 Wochen

Ein aus den Pressplatten des Beispiels 19 gestanzter Probekörper (100 x 100 mm, Dicke 1 mm) wurde 24 h bei 23 +/-1 °C gelagert und ausgewogen. Der Prüfkörper wurde so zwischen zwei adsorbierende Kontaktscheiben gebracht, dass ihre Achsen übereinstimmten und ein "Sandwich"-Verbund gebildet wurde. Dieser Verbund wurde mittig mit einem Gewicht von 2 kg beschwert. Der Stapel wurde in den vorgeheizten Temperierschrank (70 °C ± 1 °C) auf einer Gitterrostschiene platziert.
Der Probenkörper wurde nach 4 Wochen gewogen. Es wurde eine Doppelbestimmung (2 Probenkörper je Estergemisch) durchgeführt. Als adsorbierende Kontaktscheiben wurden High Impact Polystyrol (HIPS, Dicke: 2 mm) und Hart-PVC (Dicke: 2 mm) eingesetzt.

Tabelle 28: Migration nach 4 Wochen in Massen% (Gemische auf Basis Sojaöl)

|  | "nicht-abgereichertes" Gemisch-S | | "abgereichertes" Gemisch-S | |
|---|---|---|---|---|
|  | HIPS | Hart-PVC | HIPS | Hart-PVC |
| Methylester | 25,3 | 21,8 | 23,1 | 19,1 |
| Butylester | - | - | - | - |

(fortgesetzt)

| | "nicht-abgereichertes" Gemisch-S | | "abgereichertes" Gemisch-S | |
|---|---|---|---|---|
| | HIPS | Hart-PVC | HIPS | Hart-PVC |
| *n*-Pentylester | 26,7 | 18,7 | 23,5 | 18,7 |
| 2-Ethylhexylester | 21,7 | 11,7 | 19,6 | 14,6 |
| *iso*-Nonylester | 21,9 | 10,7 | 20,3 | 13,7 |
| 2-Propylheptylester | - | - | 18,4 | 12,2 |

Tabelle 29: Migration nach 4 Wochen in Massen-% (Gemische auf Basis Leinöl)

| | "nicht-abgereichertes" Gemisch-L | | "abgereichertes" Gemisch-L | |
|---|---|---|---|---|
| | HIPS | Hart-PVC | HIPS | Hart-PVC |
| Methylester | 23,6 | 21,3 | 18,0 | 20,6 |
| Butylester | 22,0 | 19,0 | 16,5 | 18,3 |
| *n*-Pentylester | 21,8 | 18,9 | 16,2 | 17,7 |
| 2-Ethylhexylester | 19,0 | 17,9 | 14,2 | 14,4 |
| *iso*-Nonylester | 17,5 | 13,6 | 14,6 | 14,2 |
| 2-Propylheptylester | - | - | 12,4 | 11,7 |

Erfindungsgemäße Weichmacher zeigen eine deutlich geringere Migrationsneigung in schlagzähes Polystyrol. Diese Eigenschaft ist vorteilhaft bei der Konfektionierung von Multilayersystemen.

**Patentansprüche**

1. Gemisch umfassend epoxidierte Fettsäureester, welches einen geringeren Massenanteil an Fettsäureketten-haltigen Verbindungen enthält, die in der Fettsäurekette keine funktionellen Gruppen einschließlich Mehrfachbindungen aufweisen, als das Gemisch der Fettsäureketten-haltigen Verbindungen, aus denen das Gemisch umfassend epoxidierte Fettsäureester hergestellt wurde.

2. Gemisch umfassend epoxidierte Fettsäureester nach Anspruch 1, **dadurch gekennzeichnet, dass** das Gemisch der Fettsäureketten-haltigen Verbindungen, aus denen das Gemisch umfassend epoxidierte Fettsäureester hergestellt wurde, aus einem pflanzlichen Öl oder aus einem Gemisch mehrerer pflanzlicher Öle gewonnen wurde.

3. Verfahren zur Herstellung eines Gemisches umfassend epoxidierte Fettsäureester umfassend folgende Verfahrensschritte:

   - Verringerung des Anteils der Fettsäuren beziehungsweise Fettsäureester, welche in der Fettsäurekette keine funktionellen Gruppen einschließlich Mehrfachbindungen aufweisen, vor der Epoxidierung der Fettsäure(n)/- ester ODER
   - Verringerung des Anteils der Fettsäuren beziehungsweise Fettsäureestern, welche in der Fettsäurekette keine funktionellen Gruppen einschließlich Mehrfachbindungen aufweisen, nach der Epoxidierung der Fettsäuren beziehungsweise Fettsäureester,
   - im Falle der epoxidierten Fettsäuren eine Veresterung und
   - optional im Falle der epoxidierten Fettsäureester eine Umesterung.

4. Verfahren nach Anspruch 3, **gekennzeichnet durch** folgende Schritte:

a) Gewinnung eines Fettsäuregemisches beziehungsweise Fettsäureestergemisches, vorzugsweise aus einem pflanzlichen Öl oder einem Gemisch von mindestens zwei pflanzlichen Ölen,

b) Verringerung des Anteils der Fettsäuren beziehungsweise der Fettsäureester, welche in der Fettsäurekette keine funktionellen Gruppen einschließlich Mehrfachbindungen aufweisen, im Gemisch aus Schritt a)

c) Epoxidierung des Gemisches aus Schritt b),

d) Veresterung beziehungsweise optional Umesterung des Gemisches aus Schritt c).

5. Verfahren nach Anspruch 3, **gekennzeichnet durch** folgende Schritte:

a) Gewinnung eines Fettsäuregemisches beziehungsweise Fettsäureestergemisches, vorzugsweise aus einem pflanzlichen Öl oder einem Gemisch von mindestens zwei pflanzlichen Ölen,

b) Epoxidierung des Gemisches aus Schritt a),

c) Verringerung des Anteils der Fettsäuren beziehungsweise der Fettsäureester, welche in der Fettsäurekette keine funktionellen Gruppen einschließlich Mehrfachbindungen aufweisen, im Gemisch aus Schritt b),

d) Veresterung beziehungsweise optional Umesterung des Gemisches aus Schritt c).

6. Verfahren nach einem der Ansprüche 3 bis 5, **dadurch gekennzeichnet, dass** in dem Verfahren der Massenanteil der Fettsäureketten-haltigen Verbindungen, welche in der Fettsäurekette keine funktionellen Gruppen einschließlich Mehrfachbindungen aufweisen, im Vergleich zu dem Gemisch der Fettsäureketten-haltigen Verbindungen, aus denen das Gemisch umfassend epoxidierte Fettsäureester hergestellt wurde, um mindestens 20 %, vorzugsweise um mindestens 40 % mit Vorzug dazu um mindestens 60 % und insbesondere um mindestens 80 % verringert wird.

7. Gemisch umfassend epoxidierte Fettsäureester, welches mittels eines Verfahrens gemäß einem der der Ansprüche 3 bis 6 hergestellt wurde.

8. Gemisch umfassend epoxidierte Fettsäureester nach einem der Ansprüche 1, 2 oder 7, **dadurch gekennzeichnet, dass** in dem Gemisch umfassend epoxidierte Fettsäureester der Massenanteil der Fettsäureketten-haltigen Verbindungen, welche in der Fettsäurekette keine funktionellen Gruppen einschließlich Mehrfachbindungen aufweisen, im Vergleich zu dem Gemisch der Fettsäureketten-haltigen Verbindungen, aus denen das Gemisch umfassend epoxidierte Fettsäureester hergestellt wurde, um mindestens 20 %, vorzugsweise um mindestens 40 % mit Vorzug dazu um mindestens 60 % und insbesondere um mindestens 80 % verringert ist.

9. Gemisch umfassend epoxidierte Fettsäureester nach einem der Ansprüche 1, 2, 7 oder 8, **dadurch gekennzeichnet, dass** die Alkoholatreste der Fettsäureester in dem Gemisch umfassend epoxidierte Fettsäureester ausgewählt sind aus Alkoholatresten, welche 1 bis 20, vorzugsweise 2 bis 15 und insbesondere 4 bis 11 Kohlenstoffatome enthalten, wobei die Alkoholatreste bevorzugt (neben der Alkoholatfunktion) keine weiteren funktionellen Gruppen einschließlich Mehrfachbindungen aufweisen.

10. Gemisch umfassend epoxidierte Fettsäureester nach einem der Ansprüche 1, 2, 7, 8 oder 9, **dadurch gekennzeichnet, dass** das Gemisch umfassend epoxidierte Fettsäureester weniger als 20 Massen-%, vorzugsweise weniger als 15 Massen-%, mit besonderem Vorzug weniger als 10 Massen-% und insbesondere weniger als 5 Massen-% an Verbindungen enthält, welche keine Fettsäureester sind.

11. Verwendung eines Gemisch umfassend epoxidierte Fettsäureester nach einem der Ansprüche 1, 2, 7, 8, 9 oder 10 als Weichmacher für Polymere, insbesondere für PVC.

12. Verwendung eines Gemisch umfassend epoxidierte Fettsäureester nach einem der Ansprüche 1, 2, 7, 8, 9 oder 10 zur Verbesserung der Gelierung eines Plastisols und/oder zur Verringerung der Viskosität eines Plastisols und/oder zur Verringerung der Glasübergangstemperatur eines mit dem erfindungsgemäßen Gemisch hergestellten Mittels und/oder zur Verbesserung der Thermostabilität eines mit dem erfindungsgemäßen Gemisch hergestellten Mittels.

13. Mittel enthaltend ein Gemisch umfassend epoxidierte Fettsäureester nach einem der Ansprüche 1, 2, 7, 8, 9 oder 10 sowie ein oder mehrere Polymere aus der Gruppe die gebildet wird von Polyvinylchlorid, Co-Polymere von Vinylchlorid mit Vinylacetat oder mit Butylacrylat, Polyalkylmethacrylat (PAMA), Polyvinylbutyral (PVB), Polyurethan, Polysulfide, Polymilchsäure (PLA), Polyhydroxybutyral (PHB) und Nitrocellulose.

14. Mittel nach Anspruch 13, **dadurch gekennzeichnet, dass** das Mittel weniger als 5 Massen% Phthalat-haltige Verbindungen enthält, insbesondere Phthalat-frei ist.

# EP 2 990 470 A1

**Europäisches Patentamt**
**European Patent Office**
**Office européen des brevets**

## EUROPÄISCHER RECHERCHENBERICHT

**Nummer der Anmeldung**

EP 14 18 2190

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| E | WO 2014/135366 A1 (EVONIK INDUSTRIES AG [DE]; WOLDT BENJAMIN [DE]; GRASS MICHAEL [DE]; WO) 12. September 2014 (2014-09-12) * Seite 2, Zeile 26; Anspruch 5; Tabelle 1 * <br> * Seite 14, Zeile 10 - Seite 20, Zeile 30 * <br> ----- | 1-14 | INV. C11C3/00 |
| X | WO 2011/090812 A2 (ARCHER DANIELS MIDLAND CO [US]; POLYONE CORP [US]; HAGBERG ERIK [US];) 28. Juli 2011 (2011-07-28) * Absatz [0080] - Absatz [0129]; Ansprüche 1, 20,21; Beispiele 17,18 * <br> ----- | 1-4,6-14 | |
| X | CN 102 517 157 A (INST CHEM IND FOREST PROD CAS) 27. Juni 2012 (2012-06-27) * Verkörperungsbeispiel 1 * <br> ----- | 1-3, 6-11,14 | |
| A | DE 101 04 815 A1 (COGNIS DEUTSCHLAND GMBH [DE]) 8. August 2002 (2002-08-08) * das ganze Dokument * <br> ----- | 1-14 | RECHERCHIERTE SACHGEBIETE (IPC) |
| A | WO 2007/075499 A2 (ARCHER DANIELS MIDLAND CO [US]; BINDER THOMAS P [US]; GEIER DOUG [US];) 5. Juli 2007 (2007-07-05) * das ganze Dokument * <br> ----- | 1-14 | C11C |

Der vorliegende Recherchenbericht wurde für alle Patentansprüche erstellt

| Recherchenort | Abschlußdatum der Recherche | Prüfer |
|---|---|---|
| Den Haag | 27. Februar 2015 | Villányi Kelemen, K |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

&  : Mitglied der gleichen Patentfamilie, übereinstimmendes Dokument

EPO FORM 1503 03.82 (P04C03)

33

## ANHANG ZUM EUROPÄISCHEN RECHERCHENBERICHT
## ÜBER DIE EUROPÄISCHE PATENTANMELDUNG NR.

EP 14 18 2190

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-02-2015

| Im Recherchenbericht angeführtes Patentdokument | Datum der Veröffentlichung | Mitglied(er) der Patentfamilie | Datum der Veröffentlichung |
|---|---|---|---|
| WO 2014135366 A1 | 12-09-2014 | DE 102013203973 A1<br>WO 2014135366 A1 | 11-09-2014<br>12-09-2014 |
| WO 2011090812 A2 | 28-07-2011 | CN 103119094 A<br>EP 2526147 A2<br>US 2012277357 A1<br>WO 2011090812 A2 | 22-05-2013<br>28-11-2012<br>01-11-2012<br>28-07-2011 |
| CN 102517157 A | 27-06-2012 | KEINE | |
| DE 10104815 A1 | 08-08-2002 | AT 286889 T<br>DE 10104815 A1<br>EP 1355896 A1<br>ES 2235023 T3<br>US 2004106812 A1<br>WO 02060884 A1 | 15-01-2005<br>08-08-2002<br>29-10-2003<br>01-07-2005<br>03-06-2004<br>08-08-2002 |
| WO 2007075499 A2 | 05-07-2007 | BR PI0620310 A2<br>CA 2633509 A1<br>EP 1979062 A2<br>US 2007181504 A1<br>US 2010093530 A1<br>WO 2007075499 A2 | 08-11-2011<br>05-07-2007<br>15-10-2008<br>09-08-2007<br>15-04-2010<br>05-07-2007 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0198404 A2
- WO 2011072346 A1
- US 20020013396 A1
- GB 805252 B
- US 4486561 B
- WO 2013003225 A2